(19) Europäisches Patentamt
European Patent Office
Office européen des brevets

(11) **EP 4 003 472 B1**

(12) **EUROPÄISCHE PATENTSCHRIFT**

(45) Veröffentlichungstag und Bekanntmachung des Hinweises auf die Patenterteilung:
**13.03.2024   Patentblatt 2024/11**

(21) Anmeldenummer: **20749855.1**

(22) Anmeldetag: **29.07.2020**

(51) Internationale Patentklassifikation (IPC):
***A61M 16/00*** *(2006.01)*

(52) Gemeinsame Patentklassifikation (CPC):
**A61M 16/024;** A61M 16/0066; A61M 16/0883; A61M 16/16; A61M 16/202; A61M 2016/0027; A61M 2016/0033; A61M 2205/00; A61M 2205/3331; A61M 2205/3334; A61M 2209/08; A61M 2209/084

(86) Internationale Anmeldenummer:
**PCT/EP2020/071397**

(87) Internationale Veröffentlichungsnummer:
**WO 2021/018962 (04.02.2021 Gazette 2021/05)**

(54) **SYSTEM ZUR REGELUNG EINER BEATMUNGSGRÖSSE EINES BEATMUNGSGERÄTS UND BEATMUNGSGERÄT**

SYSTEM FOR CONTROLLING A VENTILATION VARIABLE OF A VENTILATOR AND VENTILATOR

SYSTÈME DE COMMANDE D'UNE VARIABLE DE VENTILATION D'UN VENTILATEUR ET VENTILATEUR

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **30.07.2019   DE 102019120541**

(43) Veröffentlichungstag der Anmeldung:
**01.06.2022   Patentblatt 2022/22**

(73) Patentinhaber: **Hamilton Medical AG 7402 Bonaduz (CH)**

(72) Erfinder: **MEIER, Kevin 7000 Chur (CH)**

(74) Vertreter: **Schmitt-Nilson Schraud Waibel Wohlfrom Patentanwälte Partnerschaft mbB Pelkovenstraße 143 80992 München (DE)**

(56) Entgegenhaltungen:
WO-A1-98/12965       WO-A1-2005/051469
WO-A1-2016/149743    US-A1- 2007 151 563

**Beschreibung**

Die vorliegende Erfindung liegt auf dem Gebiet der Beatmungsgeräte, d.h. die vorliegende Erfindung liegt auf dem Gebiet der maschinellen Beatmung von Patienten. Insbesondere betrifft die vorliegende Erfindung die Regelung einer Beatmungsgröße, wie z.B. des Beatmungsdrucks oder des Beatmungsvolumens, eines Beatmungsgeräts.

Bei der maschinellen Beatmung wird einem Patienten Atemluft auf maschinellem Wege, in der Regel unter Aufbringung eines Überdrucks zugeführt. Die maschinelle Beatmung kann die eigene Atmung des Patienten unterstützen oder vollständig ersetzten. Das Zuführen der Beatmungsluft erfolgt in aufeinander folgenden Beatmungszyklen. Jeder Beatmungszyklus hat eine Inspirationsphase mit nachfolgender Expirationsphase. Dabei wird die Beatmungsluft während der Inspirationsphase eines jeweiligen Beatmungszyklus zugeführt, in der Regel maschinell oder jedenfalls maschinell unterstützt, und mit einem Überdruck gegenüber dem in den Atemwegen des Patienten herrschenden Druck. Das Ausatmen erfolgt während einer nachfolgenden Expirationsphase des Beatmungszyklus. Hierbei erfolgt meist keine Beaufschlagung der Atemwege mit Überdruck oder Unterdruck, vielmehr erfolgt das Ausatmen meist passiv durch Entspannen der Atemwege gegenüber dem Umgebungsdruck. Es ist aber auch möglich, dass in der Expirationsphase eine maschinelle Unterstützung erfolgt.

WO 2005/051469 A1 offenbart ein Verfahren und eine Vorrichtung zur Bereitstellung von Atmungsunterstützung für einen spontan atmenden Patienten. Es wird ein Fehlersignal berechnet, das die Differenz zwischen einer Funktion des Atemluftstroms über einen Zeitraum und einem Zielwert ist. Unter Verwendung einer Servoschleife wird dem Patienten Luft mit einem Druck zugeführt, der eine Funktion des Fehlersignals, der Phase des aktuellen Atmungszyklus und einer Schleifenverstärkung ist, die in Abhängigkeit von der Größe des Fehlersignals variiert. Die Schleifenverstärkung steigt mit der Größe des Fehlersignals, und die Verstärkung ist für Fehlersignale unterhalb eines Beatmungszielwerts größer als für Fehlersignale oberhalb des Beatmungszielwerts. Der Zielwert ist eine alveoläre Ventilation, die den physiologischen Totraum des Patienten berücksichtigt.

WO 2016/149743 A1 offenbart ein Verfahren und eine Vorrichtung zur Behandlung einer Atemwegserkrankung. Beispielsweise liefert ein Druckgenerator einen Luftstrom mit positivem Druck über eine Patientenschnittstelle in die Atemwege eines Patienten. Ein Sensor erzeugt ein Signal, das die Atmungsflussrate des Patienten darstellt. Eine Steuerung steuert den Druckgenerator, um der Patientenschnittstelle eine Beatmungstherapie mit einem Basisdruck bereitzustellen. Die Steuerung berechnet aus dem Signal ein Maß der Ventilation des Patienten. Die Steuerung berechnet aus einem Inspirationsteil des Signals ein Maß der Flussbegrenzung.

Die Steuerung berechnet ein Verhältnis des Ventilationsmaßes und einer erwarteten normalen Ventilation. Die Steuerung passt einen Sollwert für den Basisdruck der Beatmungstherapie basierend auf dem Maß der Flussbegrenzung an. Die Anpassung kann ferner von einem Vergleich zwischen dem Verhältnis und einer relativen Ventilationsschwelle abhängen, die ansteigt, wenn das Maß der Durchflussbegrenzung ansteigt.

Bei der maschinellen Beatmung wird häufig versucht, eine Beatmungsgröße, wie z.B. den Beatmungsdruck oder den Beatmungsfluss, d.h. das Beatmungsvolumen pro Zeiteinheit, in zeitlich veränderlicher Art und Weise aufzubringen. Insbesondere wird versucht, die Beatmungsgröße gemäß einer gewünschten Sollkurve für eine Inspirationsphase und/oder für eine Expirationsphase aufzubringen. Die gewünschte Sollkurve kann dabei ein Modell für das normale Atemverhalten des Patienten sein oder ein für den momentanen Zustand des Patienten besonders günstiges Atemverhalten abbilden, etc..

Das Aufbringen der Beatmungsgröße gemäß einer gewünschten Sollkurve gestaltet sich im Allgemeinen recht schwierig. Die bisherigen Ansätze zur Aufbringung der Beatmungsgröße gemäß einer gewünschten Sollkurve sind nicht immer zufriedenstellend. Insbesondere können die Ergebnisse bisheriger Ansätze für unterschiedliche Patienten stark divergieren.

Demzufolge wäre es wünschenswert, ein System und ein Verfahren zur Regelung einer Beatmungsgröße eines Beatmungsgeräts bereitzustellen, die einen weiten Anwendungsbereich für die maschinelle Beatmung erlauben. Insbesondere wäre es wünschenswert, ein System und ein Verfahren bereitzustellen, die auch bei divergierenden anatomischen Gegebenheiten der Patienten zu guten Beatmungsergebnissen führen.

Die vorliegenden Erfindung umfasst ein System zur Regelung einer Beatmungsgröße eines Beatmungsgeräts für die Nachführung entlang einer Sollkurve, aufweisend: mindestens einen Sensor, der eingerichtet ist, Momentanwerte für die Beatmungsgröße zu erfassen; einen Aktuator, der eingerichtet ist, die Beatmungsgröße des Beatmungsgeräts anzupassen; einen Speicher, in dem die Sollkurve für die Beatmungsgröße hinterlegt ist, wobei die Sollkurve für eine Inspirationsphase und/oder für eine Expirationsphase hinterlegt ist; und einen Regler, der zumindest ein Proportional-Glied und ein Integral-Glied aufweist, der eingerichtet ist, aus der Sollkurve und den Momentanwerten für die Beatmungsgröße eine Regeldifferenz zu bestimmen, und der eingerichtet ist, den Aktuator zu steuern; wobei der Regler eingerichtet ist, einen Verstärkungsfaktor des Integral-Glieds gemäß mindestens einem der folgenden Merkmale anzupassen: (i) Erhöhen des Verstärkungsfaktors des Integral-Glieds, wenn das Integral der Regeldifferenz für die Inspirationsphase und/oder die Expirationsphase einen vorbestimmten Integral-Schwellenwert überschreitet; (ii) Reduzieren des Verstärkungsfaktors des Integral-Glieds, wenn eine Anzahl von Nulldurchgängen der Regeldifferenz während der Inspirationsphase und/oder während der Expirationsphase einen vorbestimmten Nulldurchgang-Schwellenwert überschreitet.

Beispielhafte Ausführungsformen der Erfindung erlauben eine effektive und leicht zu implementierende Anpassung der Regelung der Atmungsgröße für die vorliegenden Beatmungsgegebenheiten, wie z.B. für einen zu beatmenden Patienten. Das Vorsehen eines Reglers, der zumindest ein Proportional-Glied und ein Integral-Glied aufweist, und das Anpassen des Verstärkungsfaktors des Integral-Glieds in der oben genannten Art ermöglichen eine effektive Anpassung des Reglers und somit eine effektive Nachführung der Beatmungsgröße entlang der Sollkurve. Dabei kann der Regler sein Verhalten aus sich heraus anpassen und ist nicht auf Daten bezüglich der Anatomie des Patienten angewiesen. Im Vergleich zu früheren Ansätzen, bei denen der Widerstand und die Expansionsfähigkeit des Beatmungstrakts des Patienten für die Parametrisierung des Reglers verwendet worden sind, ermöglichen beispielhafte Ausführungsformen der Erfindung eine selbsttätige Anpassung der Nachführung entlang der Sollkurve während der Beatmung. Somit kann das Bestimmen des Widerstands und der Expansionsfähigkeit des Beatmungstrakts des Patienten durch die hierin beschriebene Regelung überflüssig gemacht werden. Die inhärenten Probleme einer solchen Bestimmung von Widerstand und Expansionsfähigkeit des Beatmungstrakts des Patienten, nämlich eine hohe Komplexität und eine geringe Genauigkeit der Bestimmung der genannten Parameter, können somit ebenfalls vermieden werden. Beispielhafte Ausführungsformen der Erfindung erlauben eine Regelung der Beatmungsgröße, die eine effektive Nachführung entlang der Sollkurve ermöglicht, ohne dass im Vorhinein schwer feststellbare Patientendaten ermittelt oder modelliert werden müssen.

Der Regler ist eingerichtet, den Verstärkungsfaktor des Integral-Glieds zu erhöhen, wenn das Integral der Regeldifferenz für die Inspirationsphase und/oder die Expirationsphase einen vorbestimmten Integral-Schwellenwert überschreitet, und/ oder den Verstärkungsfaktor des Integral-Glieds zu reduzieren, wenn eine Anzahl von Nulldurchgängen der Regeldifferenz während der Inspirationsphase und/oder während der Expirationsphase einen vorbestimmten Nulldurchgang-Schwellenwert überschreitet. In anderen Worten, es ist möglich, dass der Regler lediglich eingerichtet ist, den Verstärkungsfaktor des Integral-Glieds zu erhöhen, wenn das Integral der Regeldifferenz für die Inspirationsphase und/oder die Expirationsphase einen vorbestimmten Integral-Schwellenwert überschreitet, oder dass der Regler lediglich eingerichtet ist, den Verstärkungsfaktor des Integral-Glieds zu reduzieren, wenn eine Anzahl von Nulldurchgängen der Regeldifferenz während der Inspirationsphase und/oder während der Expirationsphase einen vorbestimmten Nulldurchgang-Schwellenwert überschreitet, oder dass der Regler eingerichtet ist, den Verstärkungsfaktor des Integral-Glieds zu erhöhen, wenn das Integral der Regeldifferenz für die Inspirationsphase und/oder die Expirationsphase einen vorbestimmten Integral-Schwellenwert überschreitet, und den Verstärkungsfaktor des Integral-Glieds zu reduzieren, wenn eine Anzahl von Nulldurchgängen der Regeldifferenz während der Inspirationsphase und/oder während der Expirationsphase einen vorbestimmten Nulldurchgang-Schwellenwert überschreitet. Durch das Erhöhen des Verstärkungsfaktors des Integral-Glieds kann der Regler dahingehend angepasst werden, dass er auf Abweichungen zwischen der Beatmungsgröße und der Sollkurve, insbesondere auf Abweichungen zwischen der Beatmungsgröße und der Sollkurve, die sich über einen vergleichsweise längeren Zeitraum hinziehen, stärker und somit schneller reagiert. Das Reduzieren des Verstärkungsfaktors des Integral-Glieds kann den Regler verlangsamen und so ein Überschießen der Sollkurve, insbesondere ein starkes Schwingen der Beatmungsgröße um die Sollkurve herum, verringern bzw. verhindern.

Die Sollkurve ist in dem Speicher für eine Inspirationsphase und/oder für eine Expirationsphase hinterlegt. In anderen Worten, es ist möglich, dass die hinterlegte Sollkurve lediglich die Inspirationsphase betrifft oder dass die hinterlegte Sollkurve lediglich die Expirationsphase betrifft oder dass die hinterlegte Sollkurve sowohl die Inspirationsphase als auch die Expirationsphase betrifft. Es ist möglich, dass der Regler die Beatmungsgröße nur in der Inspirationsphase entlang einer Sollkurve nachführt oder nur in der Expirationsphase entlang einer Sollkurve nachführt oder sowohl in der Inspirationsphase als auch in der Expirationsphase entlang einer Sollkurve nachführt. Der Regler kann für die Inspirationsphase und die Expirationsphase unterschiedliche Parameter für das Proportional-Glied und das Integral-Glied aufweisen. In anderen Worten, der Regler kann die Inspirationsphase und die Expirationsphase unterschiedlich behandeln. Insbesondere kann der Verstärkungsfaktor des Integral-Glieds für die Inspirationsphase und die Expirationsphase unterschiedlich sein. Es ist aber auch möglich, dass der Regler die Inspirationsphase und die Expirationsphase gleich behandelt, d.h. für die Inspirationsphase und die Expirationsphase gleich parametrisiert ist.

Gemäß einer weiteren Ausführungsform ist die Beatmungsgröße eines von Beatmungsdruck und Beatmungsvolumen. In anderen Worten, die geregelte Beatmungsgröße ist der von dem Beatmungsgerät zur Verfügung gestellte Beatmungsdruck oder das von dem Beatmungsgerät zur Verfügung gestellte Beatmungsvolumen, d.h. das von dem Beatmungsgerät zur Verfügung gestellte Luftvolumen. Das Beatmungsvolumen kann als Beatmungsvolumen pro Zeiteinheit und somit als Beatmungsfluss bzw. Luftfluss betrachtet werden. Der mindestens eine Sensor kann mindestens einen Drucksensor und/oder mindestens einen Volumen- bzw. Flusssensor umfassen.

Gemäß einer weiteren Ausführungsform weist der Aktuator einen gesteuerten Bläser und/oder ein gesteuertes Ventil auf. Auf diese Weise kann die Beatmungsgröße durch Erzeugen von Druckluft über einen gesteuerten Bläser und/oder durch Regulieren von Druckluft über ein gesteuertes Ventil geregelt werden. Es ist möglich, die Beatmungsgröße alleine über eine einstellbare Quelle, wie z.B. über einen gesteuerten Bläser, oder über einen einstellbaren Zugang zu einer nicht einstellbaren Quelle, wie z.B. durch ein gesteuertes Ventil zu einem Luftdruck-Reservoir, oder durch die Kombination einer einstellbaren Quelle und eines einstellbaren Zugangs zu regeln. Insbesondere kann der Aktuator für die Inspirationsphase lediglich einen gesteuerten Bläser oder lediglich ein gesteuertes Inspirationsventil oder eine Kombination aus gesteuertem Bläser und gesteuertem Inspirationsventil aufweisen. Für die Expirationsphase kann der Aktuator ein gesteuertes Expirationsventil aufweisen. Es ist aber auch möglich, dass das Beatmungsgerät für die Expirationsphase ein vollkommen passives System darstellt, durch das Luft aus der Lunge des Patienten passiv in Folge des während der Inspirationsphase aufgebauten Überdrucks in der Lunge abgelassen wird. Der Aktuator kann jede beliebige Komponente bzw. jede beliebige Kombination von Komponenten aufweisen, die geeignet ist, eine gewünschte Beatmungsgröße des Beatmungsgeräts anzupassen.

Gemäß einer weiteren Ausführungsform ist der Regler ein Proportional-Integral-Regler (PI-Regler). In anderen Worten, der Regler weist nur ein Proportional-Glied und ein Integral-Glied auf. Es ist demzufolge möglich, den Regler ohne weitere Glieder, z.B. ohne Differential-Glied und/oder ohne Totzeit-Glied, auszuführen. Auf diese Weise ist eine effektive Nachführung entlang der Sollkurve mit geringer Komplexität und hoher Verarbeitungsgeschwindigkeit möglich. Ein besonders gutes Ansprechverhalten im Vergleich zu der typischen Dauer einer Inspirationsphase bzw. Expirationsphase kann erreicht werden.

Gemäß einer weiteren Ausführungsform ist der Regler eingerichtet, einen Verstärkungsfaktor des Proportional-Glieds unverändert zu lassen, wenn der Verstärkungsfaktor des Integral-Glieds angepasst wird. Die Erfinder des vorliegenden Systems zur Regelung einer Beatmungsgröße eines Beatmungsgeräts haben herausgefunden, dass überraschenderweise alleine durch die Anpassung des Integral-Glieds während der Beatmung eine effektive Nachführung entlang der Sollkurve erreicht werden kann. Insbesondere kann die Anpassung des Integral-Glieds ohne Anpassung des Proportional-Glieds erfolgen. Auf diese Weise kann der Regler in einer wenig komplexen, leicht zu implementierenden und leicht nachzuverfolgenden Art und Weise angepasst werden. Die hohe Transparenz der Anpassung des Reglers ist auch vor dem Hintergrund zu begrüßen, dass die Beatmung eines Patienten aufgrund der hohen Empfindlichkeit des Atmungstrakts im Allgemeinen ein hohes Risiko darstellt und dass eng begrenzte und gut nachvollziehbare Anpassungen des Reglers im Falle von Unregelmäßigkeiten auf Seiten des Patienten zur effektiven Fehlersuche beitragen können.

Gemäß einer weiteren Ausführungsform ist der Regler eingerichtet, den Verstärkungsfaktor des Proportional-Glieds im Betrieb generell unverändert zu lassen. In anderen Worten, der Verstärkungsfaktor des Proportional-Glieds kann auf einen vorbestimmten Wert eingestellt sein und dort bleiben. Der Verstärkungsfaktor des Proportional-Glieds kann dabei für alle Patienten gleich sein oder zu Beginn der Beatmung auf einen Patientenspezifischen Verstärkungsfaktor eingestellt werden.

Gemäß einer weiteren Ausführungsform ist der Regler eingerichtet, den Verstärkungsfaktor des Integral-Glieds auf einen Initialwert zu setzen. Auf diese Weise kann die Regelung der Beatmungsgröße auf eine wohldefinierte Weise beginnen und von einem wohldefinierten Startpunkt aus während der Beatmung auf die vorliegenden Gegebenheiten angepasst werden. Der Initialwert kann weiterhin sicherstellen, dass zu Beginn auf jeden Fall keine für den Patienten potenziell gefährliche Parametrisierung des Integral-Glieds vorliegt.

Gemäß einer weiteren Ausführungsform ist der Initialwert abhängig von mindestens einem Patientenparameter, wie z.B. Alter oder Gewicht. Insbesondere kann der Initialwert abhängig sein von leicht zugänglichen, makroskopischen Parametern des Patienten. Im Vergleich zu dem Widerstand und der Expansionsfähigkeit des Beatmungstrakts des Patienten sind Patientenparameter wie Alter oder Gewicht leicht festzustellen. Der Regler kann somit ohne große Komplexität die Regelung an einer bestimmten Patientenklasse ausrichten. Beispielsweise kann der Regler dahingehend angepasst sein, ob es sich bei dem Patienten um ein Kind oder einen Erwachsenen handelt. Weiterhin ist es möglich, aufgrund des Gewichts des Patienten eine erste grobe Abschätzung bezüglich des Lungenvolumens zu treffen, was in dem Initialwert reflektiert sein kann.

Gemäß einer weiteren Ausführungsform ist der Regler eingerichtet, den Verstärkungsfaktor des Integral-Glieds einmalig bei Betriebsbeginn auf den Initialwert zu setzen. Es ist auch möglich, den Verstärkungsfaktor des Integral-Glieds in vorbestimmten Abständen auf den Initialwert zu setzen. Auf diese Weise kann der wohldefinierte Startpunkt für die Regelung einmalig oder wiederholt eingenommen werden. Bei dem einmaligen Setzen des Verstärkungsfaktors des Integral-Glieds auf den Initialwert kann eine erreichte Anpassung des Reglers lange beibehalten werden. Durch ein wiederholtes Setzen des Verstärkungsfaktors des Integral-Glieds auf den Initialwert kann verhindert werden, dass eine fehlerhafte, ungewollte Entwicklung des Verstärkungsfaktors des Integral-Glieds lange fortbesteht. Das wiederholte Setzen des Verstärkungsfaktors des Integral-Glieds auf den Initialwert kann insbesondere dann eingesetzt werden, wenn die Anpassung des Verstärkungsfaktors des Integral-Glieds nur in eine Richtung erfolgt. In anderen Worten, das wiederholte Setzen des Verstärkungsfaktors des Integral-Glieds auf den Initialwert kann insbesondere dann eingesetzt werden, wenn der Regler nur eingerichtet ist, den Verstärkungsfaktor des Integral-Glieds zu erhöhen, wenn das Integral der Regeldifferenz für die Inspirationsphase und/oder die Expirationsphase einen vorbestimmten Integral-Schwellenwert überschreitet, oder wenn der Regler nur eingerichtet ist, den Verstärkungsfaktor des Integral-Glieds zu reduzieren, wenn eine Anzahl von Nulldurchgängen der Regeldifferenz während der Inspirationsphase und/oder während der Expirationsphase einen vorbestimmten Nulldurchgang-Schwellenwert überschreitet.

Gemäß einer weiteren Ausführungsform ist der Regler eingerichtet, den Verstärkungsfaktor des Integral-Glieds für die Nachführung entlang der Sollkurve für die momentane Inspirationsphase bzw. die momentane Expirationsphase anzupassen. In anderen Worten, der Regler kann eingerichtet sein, den Verstärkungsfaktor des Integral-Glieds anzupassen, bevor die momentane Inspirationsphase bzw. die momentane Expirationsphase endet. Auf diese Weise kann das Nachführen der Beatmungsgröße entlang der Sollkurve unmittelbar für eine laufende Inspirationsphase bzw. Expirationsphase angepasst werden. Der Verstärkungsfaktor des Integral-Glieds und somit die Parametrisierung des Reglers können so schnell angepasst werden, dass sich der Effekt der Anpassung schon in der momentanen Inspirationsphase bzw. Expirationsphase bemerkbar macht. Insbesondere kann der Verstärkungsfaktor des Integral-Glieds unmittelbar erhöht und/oder reduziert werden, sobald eine der hierin beschriebenen Bedingungen für das Erhöhen und/oder Reduzieren des Verstärkungsfaktors vorliegt. Weiter insbesondere kann das Anpassen des Verstärkungsfaktors des Integral-Glieds nach Detektieren einer der hierin genannten Bedingungen in einem Zeitrahmen erfolgen, der viel kleiner als die Dauer einer Inspirationsphase bzw. einer Expirationsphase ist. Der genannte Zeitrahmen kann kleiner als 1/10 oder kleiner als 1/50 oder kleiner als 1/100 der Dauer der Inspirationsphase bzw. der Expirationsphase sein.

Gemäß einer alternativen Ausführungsform ist der Regler eingerichtet, den Verstärkungsfaktor des Integral-Glieds für die Nachführung entlang der Sollkurve für die nächste Inspirationsphase bzw. die nächste Expirationsphase anzupassen. Auf diese Weise erfolgt die Regelung der Beatmungsgröße mit einem festen Verstärkungsfaktor des Integral-Glieds für eine gegebene Inspirationsphase bzw. Expirationsphase. Dies wiederum kann ein besonders stabiles, wenn auch möglicherweise langsameres Konvergieren der Regelung von Beatmungszyklus zu Beatmungszyklus ermöglichen.

Wie oben beschrieben, kann der Regler eingerichtet sein, den Verstärkungsfaktor des Integral-Glieds zu erhöhen, wenn das Integral der Regeldifferenz für die Inspirationsphase und/oder die Expirationsphase den vorbestimmten Integral-Schwellenwert überschreitet.

Gemäß einer weiteren Ausführungsform ist der Regler eingerichtet, das Integral der Regeldifferenz bei einem Nulldurchgang der Regeldifferenz auf null zu setzen. Auf diese Weise ist das Erhöhen des Verstärungsfaktors des Integral-Glieds darauf ausgerichtet, das Integral der Regeldifferenz bis zu einem Erreichen des Regelziels, d.h. bis zum Erreichen der Sollkurve, zu betrachten. Insbesondere kann das Integral der Regeldifferenz zwischen aufeinanderfolgenden Instanzen des Erreichens der Sollkurve betrachtet werden. Somit kann das Integral der Regeldifferenz als ein gutes Maß für die Schnelligkeit des Reglers in der Nachführung entlang der Sollkurve dienen, wobei das wiederholte Rücksetzen des Integrals auf null eine wiederholte Bewertung der Schnelligkeit des Reglers ermöglicht.

Gemäß einer weiteren Ausführungsform ist eine Mehrzahl von Integral-Schwellenwerten vorgesehen, und der Regler ist eingerichtet, den Verstärkungsfaktor des Integral-Glieds mehrfach zu erhöhen, wenn das Integral der Regeldifferenz mehrere der Mehrzahl von Integral-Schwellenwerten überschreitet. Auf diese Weise können mehrere Grade der Anpassung des Reglers vorgesehen sein, so dass der Verstärkungsfaktor des Integral-Glieds besonders gut auf die Regeldifferenz ausgerichtet sein kann bzw. besonders zielgerichtet angepasst werden kann. Auch kann auf diese Weise eine effektive Anpassung der Regelung für ein besonders schnelles Erreichen einer gewünschten Nachführung entlang der Sollkurve erreicht werden. Es ist möglich, dass der Regler während einer Inspirationsphase bzw. einer Expirationsphase mehrfach angepasst wird und so unmittelbar einem sich aufsummierenden Regelfehler entgegenwirkt. Die Mehrzahl von Integral-Schwellenwerten können Vielfache eines ersten Integral-Schwellenwerts sein. Es ist auch möglich, dass die Integral-Schwellenwerte in nicht-linearer Weise vorgesehen sind, beispielsweise auf Basis einer experimentellen Bestimmung geeigneter Integral-Schwellenwerte. Ebenso kann das mehrfache Erhöhen des Verstärkungsfaktors des Integral-Glieds in äquidistanten Schritten erfolgen oder auf Basis unterschiedlicher, geeignet definierter Schrittweiten erfolgen. Die Mehrzahl von Integral-Schwellenwerten und die zugehörigen Erhöhungen des Verstärkungsfaktors des Integral-Glieds können in Form einer Tabelle oder in Form einer Funktion oder auf jede andere geeignete Weise hinterlegt sein.

Wie oben ausgeführt, kann der Regler eingerichtet sein, den Verstärkungsfaktor des Integral-Glieds zu reduzieren, wenn die Anzahl von Nulldurchgängen der Regeldifferenz während der Inspirationsphase und/oder während der Expirationsphase den vorbestimmten Nulldurchgang-Schwellenwert überschreitet. Der vorbestimmte Nulldurchgang-Schwellenwert betrifft dabei die Anzahl von Nulldurchgängen während genau einer Inspirationsphase bzw. während genau einer Expirationsphase.

Ein Nulldurchgang kann definiert sein als ein Kreuzen der Beatmungsgröße und der Sollkurve für die Beatmungsgröße. Es ist auch möglich, dass ein Nulldurchgang definiert ist als ein Kreuzen der Beatmungsgröße und der Sollkurve für die Beatmungsgröße in Kombination mit einem Verlassen eines um die Sollkurve herum definierten Toleranzbereichs. In anderen Worten, ein Nulldurchgang kann definiert sein als eine Änderung des Vorzeichens der Regeldifferenz oder definiert sein als das Erreichen einer vorbestimmten Regeldifferenz nach einem Vorzeichenwechsel der Regeldifferenz. Durch das Definieren eines Toleranzbereichs bzw. durch das Definieren einer vorbestimmten Regeldifferenz für das Feststellen eines Nulldurchgangs kann verhindert werden, dass ein Oszillieren der Beatmungsgröße um die Sollkurve herum mit geringer Amplitude als Anlass angesehen wird, den Verstärkungsfaktor des Integral-Glieds anzupassen. In anderen Worten, ein Oszillieren der Beatmungsgröße um die Sollkurve herum kann als akzeptabel angesehen werden, solange die Amplitude dieser Oszillation um die Sollkurve herum gering bleibt.

Gemäß einer weiteren Ausführungsform ist eine Mehrzahl von Nulldurchgang-Schwellenwerten vorgesehen, und der Regler ist eingerichtet, den Verstärkungsfaktor des Integral-Glieds mehrfach zu reduzieren, wenn die Anzahl von Nulldurchgängen mehrere der Mehrzahl von Nulldurchgang-Schwellenwerten überschreitet. Auf diese Weise kann die Reduzierung des Verstärkungsfaktors des Integral-Glieds besonders zielgerichtet erfolgen. Insbesondere kann bei einer großen Anzahl von Nulldurchgängen, welche ein starkes Überschwingen der Beatmungsgröße anzeigt, eine starke Reduzierung des Verstärkungsfaktors des Integral-Glieds erfolgen. Die Mehrzahl von Nulldurchgang-Schwellenwerten und die zugehörigen Reduktionen des Verstärkungsfaktors des Integral-Glieds können in Form einer Tabelle oder in Form einer Funktion oder auf jede andere geeignete Weise hinterlegt sein.

Gemäß einer weiteren Ausführungsform ist der Regler eingerichtet, den Verstärkungsfaktor des Integral-Glieds in Merkmal (i) mit einem ersten Delta-Wert zu erhöhen. Der erste Delta-Wert kann ein vorbestimmter erster Delta-Wert sein. Insbesondere kann der erste Delta-Wert ein Absolutwert zur Erhöhung des Verstärkungsfaktors des Integral-Glieds sein oder ein Prozentualwert des oben beschriebenen Initialwerts des Verstärkungsfaktors des Integral-Glieds sein. Für den Fall, dass eine Mehrzahl von Integral-Schwellenwerten vorgesehen ist, kann der erste Delta-Wert bei dem Überschreiten eines jeden der Mehrzahl von Integral-Schwellenwerten für die Erhöhung des Verstärkungsfaktors des Integral-Glieds verwendet werden. Es ist jedoch auch möglich, bei einer Mehrzahl von Integral-Schwellenwerten eine Mehrzahl von ersten Delta-Werten zu verwenden.

Gemäß einer weiteren Ausführungsform ist der Regler eingerichtet, den Verstärkungsfaktor des Integral-Glieds in Merkmal (ii) um einen zweiten Delta-Wert zu reduzieren. Der zweite Delta-Wert kann ein vorbestimmter zweiter Delta-Wert sein. Insbesondere kann der zweite Delta-Wert ein Absolutwert zur Reduktion des Verstärkungsfaktors des Integral-Glieds sein oder ein Prozentualwert des oben beschriebenen Initialwerts des Verstärkungsfaktors des Integral-Glieds sein. Für den Fall, dass eine Mehrzahl von Nulldurchgang-Schwellenwerten vorgesehen ist, kann der zweite Delta-Wert bei dem Überschreiten eines jeden der Mehrzahl von Nulldurchgang-Schwellenwerten für die Reduktion des Verstärkungsfaktors des Integral-Glieds verwendet werden. Es ist jedoch auch möglich, bei einer Mehrzahl von Nulldurchgang-Schwellenwerten eine Mehrzahl von zweiten Delta-Werten zu verwenden.

Gemäß einer weiteren Ausführungsform ist der erste Delta-Wert von dem zweiten Delta-Wert verschieden. Insbesondere kann der erste Delta-Wert zwischen ein Mal und fünf Mal so groß sein wie der zweite Delta-Wert. Weiter insbesondere kann der erste Delta-Wert zwischen 1,5 Mal und drei Mal so groß sein wie der zweite Delta-Wert. Auf diese Weise kann eine Anpassung des Reglers für ein schnelleres Nachführen entlang der Sollkurve durch vergleichsweise hohe erste Delta-Werte erreicht werden, während ein ungewolltes Überschwingen der Sollkurve langsam durch vergleichsweise niedrige zweite Delta-Werte abgebaut wird. Durch die relativen Beträge von erstem Delta-Wert und zweitem Delta-Wert kann eine relative Wichtigkeit von schneller Anpassung des Reglers zur aggressiven Nachführung entlang der Sollkurve und Vermindern bzw. Verhindern von Überschwingen erreicht werden. Es wird betont, dass der erste Delta-Wert und der zweite Delta-Wert auch gleich sein können. Weiterhin wird betont, dass der zweite Delta-Wert auch größer als der erste Delta-Wert sein kann.

Gemäß einer weiteren Ausführungsform ist der Regler weiterhin eingerichtet, den Verstärkungsfaktor des Integral-Glieds gemäß dem folgenden Merkmal anzupassen: (iii) Reduzieren des Verstärkungsfaktors des Integral-Glieds, wenn der Momentanwert für die Beatmungsgröße einen Sicherheits-Schwellenwert übersteigt. Auf diese Weise kann verhindert werden, dass die Beatmungsgröße der Sollkurve zu aggressiv folgt und ein Überschießen der Sollkurve zu einem potenziell gefährlichen Lungendruck bei dem Patienten führt.

Gemäß einer weiteren Ausführungsform liegt der Sicherheits-Schwellenwert zwischen 1,5 mbar und 3 mbar über der Sollkurve.

Die vorliegende Erfindung umfasst weiterhin ein Beatmungsgerät, aufweisend ein System zur Regelung einer Beatmungsgröße gemäß einer der oben beschriebenen Ausführungsformen. Die zusätzlichen Merkmale, Modifikationen und Effekte, wie oben mit Bezug auf das System zur Regelung einer Beatmungsgröße eines Beatmungsgeräts beschrieben, sind auf das Beatmungsgerät analog anwendbar.

Gemäß einer weiteren Ausführungsform weist das Beatmungsgerät ein Verbindungselement und ein Patientenapplikationsstück auf, wobei das Patientenapplikationsstück an einem Patienten-seitigen Endbereich des Verbindungselements angeschlossen ist. Das Verbindungselement kann die Verbindung zwischen dem Patientenapplikationsstück und den weiteren Komponenten des Beatmungsgeräts, wie z.B. einer Druckluftquelle, einem Luftbefeuchter, einem Inspirationsventil, einem Expirationsventil, etc. bilden. Das Patientenapplikationsstück kann insbesondere ein Tubus oder eine Beatmungsmaske sein, je nach gewünschter Art der Beatmung des Patienten. Das Verbindungselement kann insbesondere ein einlumiger oder ein zweilumiger Verbindungsschlauch sein und kann abhängig von der Position des Luftauslasses für die Expirationsphase aufgebaut sein.

Gemäß einer weiteren Ausführungsform ist der Sensor an einem Beatmungsgerät-seitigen Endbereich des Verbindungselements angeordnet. Gemäß einer alternativen Ausführungsform ist der Sensor im Bereich des Anschlusses zwischen Verbindungselement und Patientenapplikationsstück angeordnet. Gemäß einer weiteren alternativen Ausführungsform ist ein erster Sensor an einem Beatmungsgerät-seitigen Endbereich des Verbindungselements und ein zweiter Sensor im Bereich des Anschlusses zwischen Verbindungselement und Patientenapplikationsstück angeordnet. Bei Verwendung eines einzelnen Sensors kann die Position je nach erforderlicher Genauigkeit, Komplexität der Positionierung und Fehleranfälligkeit gewählt werden. Während der Bereich des Anschlusses zwischen Verbindungselement und Patientenapplikationsstück näher am Patienten ist und somit potentiell eine bessere Schätzung des in der Lunge vorherrschenden Luftdrucks ermöglicht, kann eine Positionierung des Sensors an dem Beatmungsgerät-seitigen Endbereich des Verbindungselements zu einer weniger komplexen Implementierung und/oder einer geringeren Anfälligkeit für Störeinflüsse führen. Bei Verwendung von zwei Sensoren können durch einen Vergleich der gemessenen Werte Fehler im Beatmungssystem erkannt werden und/oder zusätzliche Anpassungen des Reglers auf Basis des Totvolumens zwischen den Sensoren durchgeführt werden.

Die vorliegende Erfindung umfasst weiterhin ein Verfahren zur Regelung einer Beatmungsgröße eines Beatmungsgeräts für die Nachführung entlang einer Sollkurve, aufweisend: Erfassen von Momentanwerten für die Beatmungsgröße; Bestimmen einer Regeldifferenz aus den Momentanwerten für die Beatmungsgröße und der Sollkurve für die Beatmungsgröße, wobei die Sollkurve für eine Inspirationsphase und/oder für eine Expirationsphase vorgegeben ist; Bestimmen einer Steuergröße für einen Aktuator, wobei das Bestimmen der Steuergröße gemäß einer Regelfunktion erfolgt, welche zumindest ein Proportional-Glied und ein Integral-Glied aufweist; Anpassen des Integral-Glieds der Regelfunktion gemäß mindestens einem der folgenden Merkmale: (i) Erhöhen des Verstärkungsfaktors des Integral-Glieds, wenn das Integral der Regeldifferenz für die Inspirationsphase und/oder die Expirationsphase einen vorbestimmten Integral-Schwellenwert überschreitet; (ii) Reduzieren des Verstärkungsfaktors des Integral-Glieds, wenn eine Anzahl von Nulldurchgängen der Regeldifferenz während der Inspirationsphase und/oder während der Expirationsphase einen vorbestimmten Nulldurchgang-Schwellenwert überschreitet; und Steuern des Aktuators gemäß der bestimmten Steuergröße zum Anpassen der Beatmungsgröße des Beatmungsgeräts. Die zusätzlichen Merkmale, Modifikationen und Effekte, wie oben mit Bezug auf das System zur Regelung einer Beatmungsgröße eines Beatmungsgeräts beschrieben, sind auf das Verfahren zur Regelung einer Beatmungsgröße eines Beatmungsgeräts analog anwendbar.

Gemäß einer weiteren Ausführungsform erfolgt das Steuern des Aktuators gemäß der bestimmten Steuergröße zum Anpassen des Beatmungsdrucks oder des Beatmungsvolumens des Beatmungsgeräts.

Gemäß einer weiteren Ausführungsform weist der Aktuator einen gesteuerten Bläser und/oder ein gesteuertes Ventil auf, und das Verfahren weist das Steuern des gesteuerten Bläsers und/oder des gesteuerten Ventils auf.

Gemäß einer weiteren Ausführungsform besteht die Regelfunktion aus einem Proportional-Glied und einem Integral-Glied.

Gemäß einer weiteren Ausführungsform lässt das Verfahren einen Verstärkungsfaktor des Proportional-Glieds unverändert, wenn der Verstärkungsfaktor des Integral-Glieds angepasst wird.

Gemäß einer weiteren Ausführungsform lässt das Verfahren den Verstärkungsfaktor des Proportional-Glieds im Betrieb generell unverändert.

Gemäß einer weiteren Ausführungsform umfasst das Verfahren weiterhin: Setzen des Verstärkungsfaktors des Integral-Glieds auf einen Initialwert.

Gemäß einer weiteren Ausführungsform erfolgt das Setzen des Verstärkungsfaktors des Integral-Glieds abhängig von mindestens einem Patientenparameter, wie z.B. Alter oder Gewicht.

Gemäß einer weiteren Ausführungsform erfolgt das Setzen des Verstärkungsfaktors des Integral-Glieds auf den Initialwert einmalig bei Betriebsbeginn oder in vorbestimmten Abständen.

Gemäß einer weiteren Ausführungsform erfolgt das Anpassen des Verstärkungsfaktors des Integral-Glieds für die Nachführung entlang der Sollkurve für die momentane Inspirationsphase bzw. die momentane Expirationsphase.

Gemäß einer weiteren Ausführungsform umfasst das Verfahren weiterhin: auf null Setzen des Integrals der Regeldifferenz bei einem Nulldurchgang der Regeldifferenz.

Gemäß einer weiteren Ausführungsform ist eine Mehrzahl von Integral-Schwellenwerten vorgesehen, und das Anpassen des Integral-Glieds der Regelfunktion umfasst: mehrfaches Erhöhen des Verstärkungsfaktors des Integral-Glieds, wenn das Integral der Regeldifferenz mehrere der Mehrzahl von Integral-Schwellenwerten überschreitet.

Gemäß einer weiteren Ausführungsform ist eine Mehrzahl von Nulldurchgang-Schwellenwerten vorgesehen, und das Anpassen des Integral-Glieds der Regelfunktion umfasst: mehrfaches Reduzieren des Verstärkungsfaktors des Integral-Glieds, wenn die Anzahl von Nulldurchgängen mehrere der Mehrzahl von Nulldurchgang-Schwellenwerten überschreitet.

Gemäß einer weiteren Ausführungsform umfasst das Anpassen des Integral-Glieds der Regelfunktion: Erhöhen des Verstärkungsfaktors des Integral-Glieds in Merkmal (i) mit einem ersten Delta-Wert und/oder Reduzieren des Verstärkungsfaktors des Integral-Glieds in Merkmal (ii) um einen zweiten Delta-Wert.

Gemäß einer weiteren Ausführungsform ist der erste Delta-Wert von dem zweiten Delta-Wert verschieden.

Gemäß einer weiteren Ausführungsform umfasst das Anpassen des Integral-Glieds der Regelfunktion: (iii) Reduzieren des Verstärkungsfaktors des Integral-Glieds, wenn der Momentanwert für die Beatmungsgröße die Sollkurve um einen Sicherheits-Schwellenwert übersteigt.

Gemäß einer weiteren Ausführungsform liegt der Sicherheits-Schwellenwert zwischen 1,5 mbar und 3 mbar.

Die vorliegende Erfindung umfasst weiterhin ein Computerprogramm bzw. ein Computerprogrammprodukt, welches Programmanweisungen enthält, die bei Ausführung auf einer Datenverarbeitungsanlage ein Verfahren gemäß einer der oben beschriebenen Ausführungsformen durchführen. Dabei können die einzelnen Schritte des Verfahrens durch die Programmanweisungen veranlasst werden und durch andere Komponenten ausgeführt werden oder in der Datenverarbeitungsanlage selbst ausgeführt werden.

Weitere beispielhafte Ausführungsformen der Erfindung werden unten mit Bezug auf die beiliegenden Figuren beschrieben.

Fig. 1 zeigt ein Beatmungsgerät zur maschinellen Beatmung von Patienten gemäß einer beispielhaften Ausführungsform der Erfindung, wobei das Beatmungsgerät mit einem System zur Regelung einer Beatmungsgröße gemäß einer beispielhaften Ausführungsform der Erfindung ausgestattet ist;

Fig. 2 zeigt ein System zur Regelung einer Beatmungsgröße eines Beatmungsgeräts gemäß einer beispielhaften Ausführungsform der Erfindung in einem Blockdiagramm;

Fig. 3 illustriert den Betrieb des Systems der Fig. 2 an Hand einer beispielhaften Sollkurve für die Beatmungsgröße und eines beispielhaften Verlaufs der Momentanwerte für die Beatmungsgröße;

Fig. 4 illustriert den Betrieb des Systems der Fig. 2 an Hand einer beispielhaften Sollkurve für die Beatmungsgröße und eines weiteren beispielhaften Verlaufs der Momentanwerte für die Beatmungsgröße;

Fig. 5 illustriert den Betrieb des Systems der Fig. 2 an Hand einer beispielhaften Sollkurve für die Beatmungsgröße und eines weiteren beispielhaften Verlaufs der Momentanwerte für die Beatmungsgröße;

Fig. 6 illustriert den Betrieb des Systems der Fig. 2 über mehrere Beatmungszyklen hinweg.

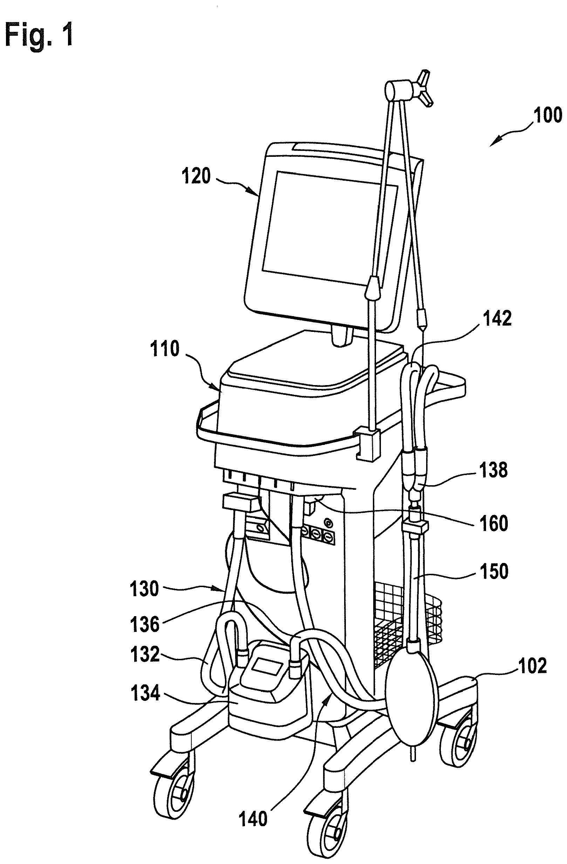

Fig. 1 zeigt ein Beatmungsgerät 100 zur maschinellen Beatmung von Patienten gemäß einer beispielhaften Ausführungsform der Erfindung. Das Beatmungsgerät 100 weist ein auf Rollen fahrbares Gestell 102 auf, an dem eine Steuereinheit 110 und ein Monitor 120 sowie eine erste Leitung 130 für von der Maschine zugeführte Beatmungsluft und eine zweite Leitung 140 für ausgeatmete Luft vorgesehen sind. Die erste Leitung 130 für zugeführte Beatmungsluft umfasst einen von der Steuereinheit 110 ausgehenden ersten Beatmungsluftschlauch 132, der zu einer Befeuchtungseinheit 134 führt, wo die Beatmungsluft durch ein Wasserreservoir geleitet wird. Von der Befeuchtungseinheit 134 führt ein zweiter Beatmungsluftschlauch 136 zu einem T-Stück 138. Von dem T-Stück gelangt die von der Beatmungsmaschine 100 während einer Inspirationsphase des Beatmungszyklus bereitgestellte Beatmungsluft über ein Patientenapplikationsstück 150 zum Patienten. Über das Patientenapplikationsstück 150 gelangt auch die vom Patienten während einer Expirationsphase des Beatmungszyklus ausgeatmete Luft zurück in Richtung Beatmungsgerät 100. Hierzu zweigt von dem T-Stück 138 ein weiterer Atemluftschlauch 142 ab, der zu der zweiten Leitung 140 für ausgeatmete Luft gehört. Der Atemluftschlauch 142 führt zu einem Expirationsventil 160, durch welches die ausgeatmete Luft in die Umgebung des Beatmungsgeräts abgegeben wird.

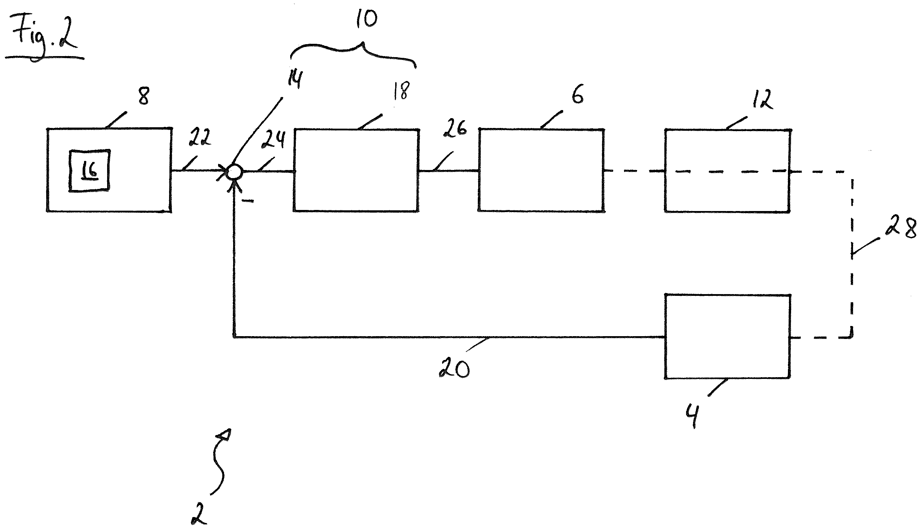

In das Beatmungsgerät 100 ist ein System zur Regelung einer Beatmungsgröße gemäß einer beispielhaften Ausführungsform der Erfindung eingebettet. Die einzelnen Komponenten dieses Systems zur Regelung einer Beatmungsgröße werden unten mit Bezug auf Fig. 2 beschrieben, wobei bezüglich einer beispielhaften Positionierung der Komponenten nochmals auf die Fig. 1 Bezug genommen wird.

Fig. 2 zeigt ein System 2 zur Regelung einer Beatmungsgröße eines Beatmungsgeräts gemäß einer beispielhaften Ausführungsform der Erfindung in einem Blockdiagramm.

Das System 2 weist einen Speicher 8 auf, in dem eine Sollkurve 16 für eine Beatmungsgröße hinterlegt ist. In der beispielhaften Ausführungsform der Fig. 2 ist die Beatmungsgröße der von dem Beatmungsgerät zur Verfügung gestellte Beatmungsdruck. Demzufolge ist die Sollkurve 16 eine Abfolge von Beatmungsdruckwerten über die Zeit. Insbesondere kann die Sollkurve 16 aus einer Abfolge von Beatmungsdruckwerten über eine Inspirationsphase und/oder über eine Expirationsphase eines Beatmungszyklus bestehen. Die Sollkurve 16 stellt einen gewünschten Verlauf des Beatmungsdrucks über die Zeit dar. Die Sollkurve 16 wird als Abfolge von Solldruckwerten von dem Speicher 8 ausgegeben.

Die Sollkurve 16 wird insbesondere als Signal 22 von dem Speicher 8 ausgegeben, wobei das Signal 22 eine Abbildung der Sollkurve 16 darstellt.

Das System 2 weist einen Sensor 4 auf, der dazu eingerichtet ist, die Beatmungsgröße zu erfassen. In der beispielhaften Ausführungsform der Fig. 2 ist der Sensor 4 ein Drucksensor. Der Sensor 4 erfasst den Beatmungsdruck 28 und gibt Momentanwerte 20 für den Beatmungsdruck aus. Der Beatmungsdruck 28 ist als physikalische Größe durch eine gestrichelte Linie in Fig. 2 dargestellt, während die Momentanwerte 20 für den Beatmungsdruck eine signalmäßige Darstellung der physikalischen Größe sind und durch eine durchgezogene Linie in Fig. 2 dargestellt sind.

Das die Sollkurve 16 repräsentierende Signal 22 und die Momentanwerte 20 des Beatmungsdrucks werden einem Subtrahierer 14 zugeführt, der aus der Sollkurve 16 und den Momentanwerten 20 für den Beatmungsdruck eine Regeldifferenz 24 bildet. Dabei subtrahiert der Subtrahierer 14 jeweils einen Momentanwert des Beatmungsdrucks von einem momentanen Sollwert aus der Sollkurve. Durch wiederholtes Subtrahieren ergibt sich ein zeitlicher Verlauf der Regeldifferenz 24 und somit ein zeitlicher Verlauf der Abweichung zwischen Sollkurve 16 und den Momentanwerten 20 des Beatmungsdrucks.

Der Subtrahierer 14 ist Teil eines Reglers 10 des Systems 2. Neben dem Subtrahierer 14 weist der Regler 10 eine Regelfunktion 18 auf, die auch als Regelalgorithmus 18 bezeichnet werden kann. Die Regeldifferenz 24 wird der Regelfunktion 18 zur Verfügung gestellt, welche mit dem Subtrahierer 14 verbunden ist. Über die Zeit wird der Regelfunktion 18 ein zeitlicher Verlauf der Regeldifferenz 24 zur Verfügung gestellt. Die Regelfunktion 18 nutzt den zeitlichen Verlauf der Regeldifferenz 24, um eine Steuergröße 26 zu erstellen und einen Aktuator 6 mittels der Steuergröße 26 zu veranlassen, auf den Beatmungsdruck 28 einzuwirken.

Der Regler 10 hat ein Proportional-Glied und ein Integral-Glied. In der beispielhaften Ausführungsform der Fig. 2 hat der Regler 10 nur ein Proportional-Glied und ein Integral-Glied. Der Regler 10 ist somit ein PI-Regler in der beispielhaften Ausführungsform der Fig. 2. Der Regler 10 kann nach der folgenden Formel arbeiten:

$$u(t) = K_P * e(t) + K_I * \int_{t0}^{t} e(\tau)\, d\tau \quad .$$

Dabei bezeichnet u(t) die Steuergröße 26 und e(t) die Regeldifferenz 24. Der lineare Term vor dem "+"-Zeichen ist das Proportional-Glied des Reglers 10, wobei $K_P$ den Verstärkungsfaktor des Proportional-Glieds bezeichnet. Der Integral-Term nach dem "+"-Zeichen ist das Integral-Glied des Reglers 10, wobei $K_I$ den Verstärkungsfaktor des Integral-Glieds bezeichnet.

Es wird betont, dass der Regler 10 auch nach einer anderen Formel arbeiten kann. Der Regler 10 hat jedoch stets ein Proportional-Glied, das die momentane Regeldifferenz berücksichtigt, sowie ein Integral-Glied, das die Regeldifferenz über einen bestimmten bzw. bestimmbaren Zeithorizont in der Vergangenheit berücksichtigt. Somit fließt die kumulierte Regeldifferenz über diesen Zeithorizont in die Regelung ein. Der Zeithorizont kann eine bestimmte Zeitspanne oder einen Zeitraum seit einem bestimmten Ereignis, z.B. den Zeitraum seit Beginn des momentanen Beatmungszyklus bzw. seit Beginn der momentanen Inspirationsphase oder Expirationsphase, umfassen.

Der Aktuator 6 passt den Beatmungsdruck 28 auf Basis der Steuergröße 26 an. Die Steuergröße 26 kann dem Aktuator 6 anzeigen, den Beatmungsdruck 28 zu erhöhen oder zu senken oder gleich zu lassen. Außerdem kann die Steuergröße 26 dem Aktuator 6 anzeigen, wie stark der Beatmungsdruck angepasst werden soll.

In der beispielhaften Ausführungsform der Fig. 2 ist der Aktuator 6 eine Druckluftquelle. Dazu kann der Aktuator 6 beispielsweise eine gesteuerte Druckluftpumpe bzw. einen gesteuerten Bläser bzw. einen gesteuerten Ventilator aufweisen. Es ist auch möglich, dass der Aktuator 6 ein Druckluftreservoir und ein gesteuertes Ventil aufweist. In jedem Fall ist der Aktuator 6 in der Lage, gesteuert Druckluft in Richtung des zu beatmenden Patienten auszugeben.

Der Aktuator 6 ist eingerichtet, den Beatmungsdruck 28 anzupassen. Für ein gegebenes Aufbringen von Druckluft durch den Aktuator 6 können sich sehr unterschiedliche Ergebnisse im Beatmungsdruck 28 einstellen. Grund dafür ist der Beatmungstrakt 12 des zu beatmenden Patienten, der von Mensch zu Mensch unterschiedlich ist. Insbesondere hat der Beatmungstrakt 12 eines jeden Patienten einen bestimmten Widerstand gegenüber der aufgebrachten Druckluft und eine bestimmte Expansionsfähigkeit des Beatmungstrakts 12 bei Aufbringung von Druckluft. Durch die Unterschiedlichkeit von Widerstand und Expansionsfähigkeit des Beatmungstrakts 12 kann die Reaktion eines Patienten auf die durch den Aktuator 6 aufgebrachte Druckluft stark variieren. Diese Zusammenhänge sind in Fig. 2 dadurch illustriert, dass der Beatmungsdruck 28 als eine gestrichelte Linie dargestellt ist, die mit dem Aktuator 6 verbunden ist und sich durch den Beatmungstrakt 12 erstreckt. Der Beatmungsdruck 28 wird durch die von dem Aktuator 6 ausgegebene Druckluft und durch die Charakteristika des Beatmungstrakts 12 beeinflusst. Der resultierende Beatmungsdruck 28 wird durch den Sensor 4 gemessen, wie durch die Verbindung der genannten gestrichelten Linie mit dem Sensor 4 veranschaulicht ist.

In dem Beatmungsgerät 100 der Fig. 1 können der Speicher 8 und der Regler 10 in der Steuereinheit 110 angeordnet sein. Auch der Aktuator 6 kann in der Steuereinheit 110 angeordnet sein. Der Sensor 4 kann in dem Beatmungsgerät-seitigen Endbereich der ersten Leitung 130 oder in dem Bereich des T-Stücks 138 oder an einer anderen geeigneten Stelle zwischen Steuereinheit 110 und Patientenapplikationsstück 150 angeordnet sein. Die Position des Sensors 4 kann je nach Anforderungen an die Distanz zwischen Sensor 4 und Patient und/oder je nach Anforderungen an die Signalübertragung zwischen Sensor 4 und Steuereinheit 110 gewählt werden. Auch kann die Position des Sensors 4 davon abhängen, ob die Regelung des Beatmungsdrucks nur für die Inspirationsphase oder nur für die Expirationsphase oder für die Inspirationsphase und die Expirationsphase stattfindet.

Der Regler 10 kann in Hardware ausgeführt sein oder als auf einem Prozessor auszuführende Software ausgeführt sein oder als Kombination von Hardware und Software ausgeführt sein.

Neben der oben beschriebenen Steuerung des Aktuators 6 hat der Regler 10 der beispielhaften Ausführungsform der Fig. 2 eine weitere, die Regelung des Beatmungsdrucks 28 beeinflussende Funktionalität. Der Regler 10 ist eingerichtet, sein Integral-Glied auf Basis der Regeldifferenz 24 anzupassen. Somit ist der Regler 10 eingerichtet, sich selbst auf Basis der Regeldifferenz 24 anzupassen. Der Regler 10 kann dadurch den Einfluss auf die Steuerung des Aktuators 6 und den Einfluss auf den Beatmungsdruck 28 auf Basis der Regeldifferenz ändern. Die Steuergröße 26 ist somit nicht nur von der Historie der Regeldifferenz 24, sondern auch von der Anpassung des Regelalgorithmus 18 innerhalb des Reglers 10 auf Basis der Historie der Regeldifferenz 24 abhängig. Auf diese Weise kann der Regler 10 sehr flexibel auf die Gegebenheiten der vorliegenden Beatmung reagieren. Insbesondere kann der Regler 10 durch die Anpassung des eigenen Regelalgorithmus 18 eine implizite Anpassung der Regelung an den Beatmungstrakt 12 des zu beatmenden Patienten erreichen.

In der beispielhaften Ausführungsform der Fig. 2 ist der Regler 10 eingerichtet, den Verstärkungsfaktor $K_I$ des Integral-Glieds zu erhöhen, wenn das Integral der Regeldifferenz 24 einen vorbestimmten Integral-Schwellenwert überschreitet.

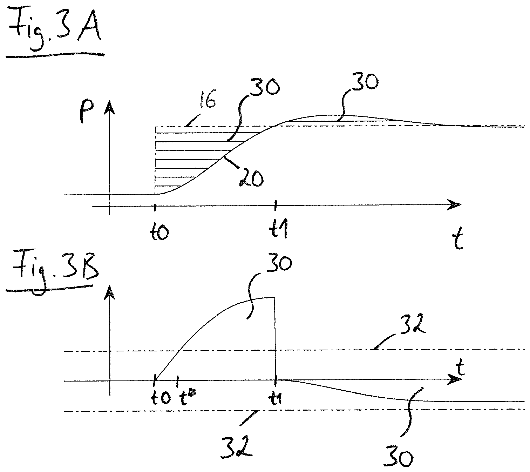

Weiterhin ist der Regler 10 eingerichtet, den Verstärkungsfaktor $K_I$ des Integral-Glieds zu reduzieren, wenn eine Anzahl von Nulldurchgängen der Regeldifferenz 24 einen vorbestimmten Nulldurchgang-Schwellenwert überschreitet. Noch weiterhin ist der Regler 10 eingerichtet, den Verstärkungsfaktor K, des Integral-Glieds zu reduzieren, wenn der Momentanwert 20 für die Beatmungsgröße die Sollkurve 16 um einen Sicherheits-Schwellenwert überschreitet. In der beispielhaften Ausführungsform der Fig. 2 sind alle drei genannten Kriterien zur Anpassung des Verstärkungsfaktors $K_I$ des Integral-Glieds implementiert. Es ist aber auch möglich, dass lediglich eines der drei genannten Kriterien oder eine Untermenge der genannten Kriterien zur Anpassung des Verstärkungsfaktors $K_I$ des Integral-Glieds implementiert ist. Die drei genannten Kriterien werden unten mit Bezug auf Fig. 3 bis 5 näher erläutert.

In der beispielhaften Ausführungsform der Fig. 2 betrachtet der Regler 10 jede Inspirationsphase bzw. Expirationsphase isoliert für die Anpassung des Verstärkungsfaktors $K_I$ des Integral-Glieds. In anderen Worten, das Integral der Regeldifferenz 24 bzw. die Anzahl der Nulldurchgänge der Regeldifferenz 24 wird am Ende einer Inspirationsphase bzw. am Ende einer Expirationsphase auf null gesetzt. Weiterhin wird das Integral der Regeldifferenz 24 auch innerhalb einer Inspirationsphase bzw. innerhalb einer Expirationsphase bei einem Nulldurchgang der Regeldifferenz 24 auf null gesetzt. Es ist aber auch möglich, dass der Regler 10 das Integral der Regeldifferenz 24 bzw. die Anzahl der Nulldurchgänge der Regeldifferenz 24 über einen längeren Zeitraum bestimmt und auf Basis dieser Bestimmung den Verstärkungsfaktor K, des Integral-Glieds anpasst.

Fig. 3 zeigt einen beispielhaften Verlauf der Momentanwerte 20 einer Beatmungsgröße für einen sprunghaften Anstieg der Sollkurve 16 der Beatmungsgröße sowie ein dazugehöriges Integral 30 der Regeldifferenz. Fig. 3 illustriert den Betrieb des Systems 2 der Fig. 2 für eine beispielhafte Sollkurve 16 und einen beispielhaften Verlauf der Momentanwerte 20 der Beatmungsgröße.

In Fig. 3A sind die Sollkurve 16 für den Beatmungsdruck 28 sowie der Verlauf der Momentanwerte 20 für den Beatmungsdruck 28 über die Zeit aufgetragen. In dem veranschaulichenden Beispiel von Fig. 3A macht die Sollkurve 16 einen positiven Drucksprung zum Zeitpunkt t0. Der positive Drucksprung zum Zeitpunkt t0 ist geeignet, das Verhalten des Reglers 10 zu Beginn einer Inspirationsphase zu veranschaulichen. Es ist für den Fachmann ersichtlich, dass die Sollkurve 16 zu Beginn der Inspirationsphase einen anderen Anstieg als den in Fig. 3A gezeigten harten Sprung haben kann.

Der Regler 10 reagiert auf den Drucksprung der Sollkurve 16 zum Zeitpunkt t0 und wirkt darauf hin, den Beatmungsdruck 28 der Sollkurve 16 nachzuführen. Fig. 3A zeigt ein Beispiel für eine sogenannte Sprungantwort des Reglers 10. Wie in Fig. 3A veranschaulicht, steigen in dem Beispiel der Fig. 3A die Momentanwerte 20 für den Beatmungsdruck 28 nur langsam an, verglichen mit dem sprunghaften Anstieg der Sollkurve 16. Der Regler 10 kann den Beatmungsdruck 28 nur langsam der Sollkurve 16 nachführen. In Fig. 3A ist das Integral 30 der Regeldifferenz 24, d.h. das Integral 30 der Differenz zwischen Sollkurve 16 und Momentanwerten 20 des Beatmungsdrucks 28 als schraffierte Fläche eingezeichnet. Bis zum Zeitpunkt t1, an dem der Momentanwert 20 des Beatmungsdrucks erstmalig die Sollkurve 16 erreicht, hat das Integral 30 der Regeldifferenz 24 einen vergleichsweise hohen Wert erreicht.

In Fig. 3B ist das Integral 30 der Regeldifferenz 24, welches in Fig. 3A als schraffierte Fläche eingezeichnet ist, als Kurve gegenüber der Zeit aufgetragen. Zum Zeitpunkt t0 ist das Integral 30 gleich null, und zum Zeitpunkt t1 wird das Integral 30 wieder auf null gesetzt. Im Betrieb wird das Integral 30 der Regeldifferenz 24 mit einem Integral-Schwellenwert 32 verglichen. Zwischen t0 und t1 gibt es einen Zeitpunkt t*, an dem das Integral 30 der Regeldifferenz 24 den Integral-Schwellenwert 32 überschreitet.

Als Folge des Überschreitens des Integral-Schwellenwerts 32 erhöht der Regler 10 den Verstärkungsfaktor $K_I$ des Integral-Glieds. Auf diese Weise reagiert der Regler 10 ab dem Zeitpunkt t* stärker auf eine kumulierte Regeldifferenz. Dies wiederum ermöglicht ein schnelleres Nachführen des Beatmungsdrucks 28 entlang der Sollkurve 16 für die Zukunft.

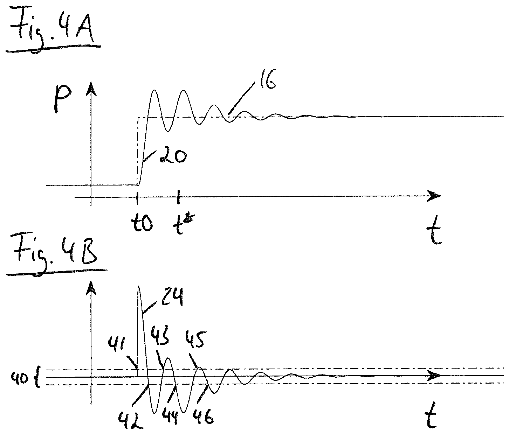

Fig. 4 zeigt einen weiteren beispielhaften Verlauf der Momentanwerte 20 einer Beatmungsgröße für einen sprunghaften Anstieg der Sollkurve 16 der Beatmungsgröße sowie einen dazugehörigen Verlauf der Regeldifferenz. Fig. 4 illustriert den Betrieb des Systems 2 der Fig. 2 für eine beispielhafte Sollkurve 16 und einen weiteren beispielhaften Verlauf der Momentanwerte 20 der Beatmungsgrö-βe.

In Fig. 4A sind die Sollkurve 16 für den Beatmungsdruck 28 sowie der Verlauf der Momentanwerte 20 für den Beatmungsdruck 28 über die Zeit aufgetragen. In dem veranschaulichenden Beispiel von Fig. 4A macht die Sollkurve 16 einen positiven Drucksprung zum Zeitpunkt t0. Wiederum ist der positive Drucksprung zum Zeitpunkt t0 geeignet, das Verhalten des Reglers 10 zu Beginn eines Inspirationsphase zu veranschaulichen. Es ist für den Fachmann ersichtlich, dass die Sollkurve 16 zu Beginn der Inspirationsphase einen anderen Anstieg als den in Fig. 4A gezeigten harten Sprung haben kann.

Der Regler 10 reagiert auf den Drucksprung der Sollkurve 16 zum Zeitpunkt t0 und wirkt darauf hin, den Beatmungsdruck 28 der Sollkurve 16 nachzuführen. Fig. 4A zeigt ein weiteres Beispiel für eine sogenannte Sprungantwort des Reglers 10. Im Gegensatz zu der Situation, wie sie in Fig. 3A veranschaulicht ist, ist der Regler 10 in der Situation der Fig. 4A imstande, den Beatmungsdruck 28 der Sollkurve 16 viel schneller nachzuführen. Der Momentanwert 20 des Beatmungsdrucks 28 erreicht die Sollkurve 16 schon kurz nach dem Zeitpunkt t0. Allerdings ist der Anstieg des Beatmungsdrucks 28 so schnell, dass der Beatmungsdruck 28 die Sollkurve 16 überschießt. Der Verlauf der Momentanwerte 20 des Beatmungsdrucks 28 kreuzt die Sollkurve sehr steil und überschießt die Sollkurve 16 deutlich. Der Regler 10 reagiert auf das Überschießen der Sollkurve 16 und wirkt auf eine Reduzierung des Beatmungsdrucks 28 hin. Als Folge überschießt der Verlauf der Momentanwerte 20 des Beatmungsdrucks 28 die Sollkurve 16 nach unten. Der Versuch des Reglers 10, den Beatmungsdruck 28 auf die Sollkurve 16 zu regeln, resultiert in einem recht hochfrequenten Oszillieren des Verlaufs der Momentanwerte 20 des Beatmungsdrucks 28 um die Sollkurve 16 herum.

Fig. 4B zeigt die Regeldifferenz 24, d.h. die Differenz zwischen Sollkurve 16 und Momentanwerten 20 des Beatmungsdrucks 28, gegenüber der Zeit. Weiterhin zeigt Fig. 4B einen Toleranzbereich 40 für die Regeldifferenz 24 um den Wert 0 herum. In der beispielhaften Ausführungsform der Fig. 4 wird ein Nulldurchgang der Regeldifferenz 24 definiert als ein Ausgehen von dem Wert null bzw. ein Queren des Werts null und ein anschließendes Verlassen des Toleranzbereichs 40. Auf Basis dieser Definition hat die beispielhafte Regeldifferenz 24 der Fig. 4 sechs Nulldurchgänge 41, 42, 43, 44, 45 und 46.

In der beispielhaften Ausführungsform der Fig. 4 ist ein Nulldurchgang-Schwellenwert von drei vorgesehen. Zum Zeitpunkt t*, wie in Fig. 4A eingezeichnet, durchläuft die Regeldifferenz 42 den vierten Nulldurchgang 44 und hat somit den Nulldurchgang-Schwellenwert von drei überschritten. Es ist auch möglich, dass der Begriff des Überschreitens des Nulldurchgang-Schwellenwerts das Erreichen des Nulldurchgang-Schwellenwerts umfasst. In diesem Fall wäre der Zeitpunkt t* schon zum Zeitpunkt des dritten Nulldurchgangs 43.

Als Folge des Überschreitens des Nulldurchgang-Schwellenwerts reduziert der Regler 10 den Verstärkungsfaktor $K_I$ des Integral-Glieds. Auf diese Weise reagiert der Regler 10 ab dem Zeitpunkt t* weniger stark auf eine kumulierte Regeldifferenz. Dies wiederum ermöglicht ein verlangsamtes Nachführen des Beatmungsdrucks 28 entlang der Sollkurve 16 für die Zukunft und somit ein weniger starkes Überschießen und Oszillieren um die Sollkurve 16.

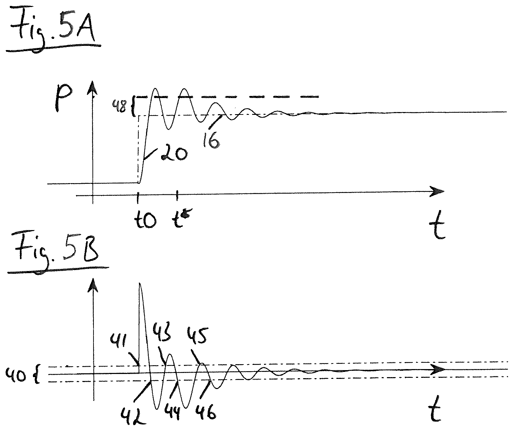

Fig. 5 zeigt einen weiteren beispielhaften Verlauf der Momentanwerte 20 einer Beatmungsgröße für einen sprunghaften Anstieg der Sollkurve 16 der Beatmungsgröße sowie einen dazugehörigen Verlauf der Regeldifferenz. Fig. 5 illustriert den Betrieb des Systems 2 der Fig. 2 für eine beispielhafte Sollkurve 16 und einen weiteren beispielhaften Verlauf der Momentanwerte 20 der Beatmungsgrö-βe.

Fig. 5 ist identisch zu Fig. 4 mit der Ausnahme, dass zusätzlich zu der Sollkurve 16 und zusätzlich zu dem Verlauf der Momentanwerte 20 der Beatmungsgröße ein Sicherheits-Schwellenwert 48 vorgesehen ist. In der beispielhaften Ausführungsform der Fig. 5 ist der Sicherheits-Schwellenwert 48 definiert als ein inkrementeller Wert gegenüber der Sollkurve 16. Der Sicherheits-Schwellenwert kann auch als Absolutwert oder auf jede andere geeignete Art und Weise definiert sein.

Der Regler 10 ist eingerichtet, den Verstärkungsfaktor K, des Integral-Glieds zu reduzieren, wenn der Verlauf der Momentanwerte 20 den Sicherheits-Schwellenwert 48 übersteigt, d.h. wenn der Verlauf der Momentanwerte 20 die Sollkurve 16 um mehr als den Sicherheits-Schwellenwert 48 übersteigt. Auf diese Weise kann ein Überschießen der Momentanwerte 20 des Beatmungsdrucks über die Sollkurve 16 vermindert werden und ein Erreichen potentiell gefährlicher Werte für den Beatmungsdruck erschwert oder verhindert werden.

In dem beispielhaften Verlauf der Momentanwerte 20 der Fig. 5 wird der Sicherheits-Schwellenwert 48 zwei Mal überschritten. Demzufolge wird der Verstärkungsfaktor K, des Integral-Glieds zwei Mal in Folge der Überschreitung des Sicherheits-Schwellenwerts 48 und ein Mal in Folge der Überschreitung des Nulldurchgang-Schwellenwerts reduziert. Es finden also insgesamt drei Reduktionen des Verstärkungsfaktors $K_I$ des Integral-Glieds statt.

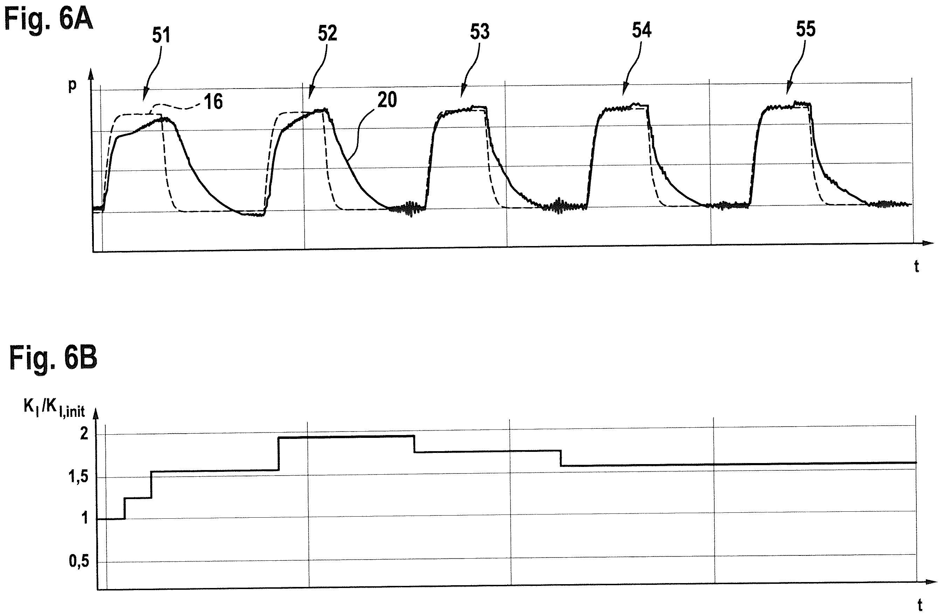

Fig. 6 zeigt einen weiteren beispielhaften Verlauf der Momentanwerte 20 einer Beatmungsgröße für eine weitere beispielhafte Sollkurve 16 der Beatmungsgrö-βe. Fig. 6 zeigt den Verlauf der Momentanwerte 20 sowie die Sollkurve 16 für eine Mehrzahl von Beatmungszyklen und zeigt die dazugehörige Anpassung des Verstärkungsfaktors des Integral-Glieds des Reglers. Fig. 6 illustriert den Betrieb des Systems 2 der Fig. 2 für eine weitere beispielhafte Sollkurve 16 und einen weiteren beispielhaften Verlauf der Momentanwerte 20 der Beatmungsgröße.

Fig. 6A zeigt fünf Beatmungszyklen 51, 52, 53, 54 und 55, von denen jeder eine Inspirationsphase und eine Expirationsphase hat. Die Sollkurve 16 stellt eine gute Annäherung an eine Sollkurve dar, wie sie bei der tatsächlichen Beatmung eines Patienten verwendet wird. In der Inspirationsphase steigt der Soll-Beatmungsdruck zuerst stark an und verbleibt dann auf einem Plateau. In der Expirationsphase fällt der Soll-Beatmungsdruck zuerst stark ab und verbleibt dann auf dem sogenannten positiv-endexspiratorischen Druck (PEEP). Der positiv-endexspiratorische Druck bleibt an dem Patienten angelegt, um ein Kollabieren der Lunge bzw. einzelner Lungenbläschen zu verhindern.

In der beispielhaften Ausführungsform der Fig. 6 arbeitet der Regler 10 darauf hin, die Momentanwerte 20 des Beatmungsdrucks 28 der Sollkurve 16 in der Inspirationsphase möglichst genau nachzuführen. In der Expirationsphase findet zuerst ein passives Ablassen des Beatmungsdrucks 28 statt, d.h. der Regler 10 unterstützt die Reduktion des Beatmungsdrucks 28 nicht in aktiver Weise. Der Verlauf der Momentanwerte 20 des Beatmungsdrucks 28 geht langsamer zurück als die Sollkurve 16. Im weiteren Verlauf der Expirationsphase wirkt der Regler 10 darauf hin, dass der Beatmungsdruck 28 auf dem positiv-endexspiratorischen Druck gehalten wird.

Fig. 6B illustriert den Verstärkungsfaktor des Integral-Glieds des Reglers 10 über die Zeit. Der Verstärkungsfaktor ist in Fig. 6B als auf den Initialwert des Verstärkungsfaktors normalisierte Größe aufgezeichnet. In anderen Worten, Fig. 6B zeigt auf der y-Achse den dimensionslosen Wert für den Quotienten von momentanem Verstärkungsfaktor des Integral-Glieds zu Initialwert des Verstärkungsfaktors des Integral-Glieds, d.h. den dimensionslosen Wert für den Quotienten $K_I / K_{I,init}$.

In der Inspirationsphase des ersten Beatmungszyklus 51 liegt ein relativ hoher Wert für das Integral der Regeldifferenz 24 vor. Das ist aus der relativ großen Fläche zwischen Sollkurve 16 und Momentanwerten 20 für den Beatmungsdruck 28 ersichtlich. In der beispielhaften Ausführungsform der Fig. 6 wird der Verstärkungsfaktor der Integral-Glieds auf Grund dieses relativ hohen Werts für das Integral der Regeldifferenz 24 zwei Mal erhöht. Diese zweimalige Erhöhung des Verstärkungsfaktors des Integral-Glieds entspricht einem Überschreiten von zwei Integral-Schwellenwerten. Das heisst, dass die beispielhafte Ausführungsform der Fig. 6 eine Mehrzahl von Integral-Schwellenwerten hat und dass bei Überschreiten eines jeden der Mehrzahl von Integral-Schwellenwerten ein Erhöhen des Verstärkungsfaktors des Integral-Glieds stattfindet. Die Erhöhung erfolgt jeweils um 0,3 auf der normierten Skala der Fig. 6B.

In der Inspirationsphase des zweiten Beatmungszyklus 52 ist der Regler 10 in der Lage, die Momentanwerte 20 des Beatmungsdrucks 28 der Sollkurve 16 besser nachzuführen. Allerdings hat das Integral der Regeldifferenz 24 auch in der Inspirationsphase des zweiten Beatmungszyklus 52 einen signifikanten Wert. Dieser liegt über dem niedrigsten Integral-Schwellenwert, so dass in der Inspirationsphase des zweiten Beatmungszyklus 52 eine weitere Erhöhung des Verstärkungsfaktors des Integral-Glieds stattfindet. Auch diese Erhöhung erfolgt um 0,3 auf der normierten Skala der Fig. 6B.

In der Expirationsphase des zweiten Beatmungszyklus 52 schwingt der Verlauf der Momentanwerte 20 des Beatmungsdrucks 28 um die Sollkurve 16, insbesondere um den positiv-endexspiratorischen Druck. Dabei tritt eine Anzahl von Nulldurchgängen auf, die größer als der vorbestimmte Nulldurchgang-Schwellenwert ist. In der beispielhaften Ausführungsform von Fig. 6 ist ein Nulldurchgang ähnlich zu Fig. 4B und Fig. 5B definiert, nämlich als ein Überschwingen über einen vorbestimmten Toleranzbereich um den positiv-endexspiratorischen Druck. Es ist aus Fig. 6A ersichtlich, dass die Momentanwerte 20 des Beatmungsdrucks 28 in der Expirationsphase des zweiten Beatmungszyklus 52 vergleichsweise stark um die Sollkurve 16 schwingen. Dadurch wird der vorbestimmte Nulldurchgang-Schwellenwert überschritten. In der beispielhaften Ausführungsform der Fig. 6 wird der Verstärkungsfaktor des Integral-Glieds auf Grund des Überschreitens des vorbestimmten Nulldurchgang-Schwellenwerts reduziert. Die Reduzierung erfolgt um 0,15 auf der normierten Skala der Fig. 6B.

In der Inspirationsphase des dritten Beatmungszyklus 53 ist das Integral der Regeldifferenz so gering, dass keine Anpassung des Verstärkungsfaktors des Integral-Glieds vorgenommen wird. In der Expirationsphase des dritten Beatmungszyklus 53 schwingt der Verlauf der Momentanwerte 20 des Beatmungsdrucks 28 wiederum derart um die Sollkurve 16, dass die Anzahl von Nulldurchgängen den vorbestimmten Nulldurchgang-Schwellenwert überschreitet. Wiederum wird der Verstärkungsfaktor des Integral-Glieds um 0,15 auf der normierten Skala der Fig. 6B reduziert.

In dem vierten Beatmungszyklus 54 und dem fünften Beatmungszyklus 55 führt der Regler 10 den Beatmungsdruck so gut der Sollkurve 16 nach, dass keine weitere Anpassung des Verstärkungsfaktors des Integral-Glieds erfolgt. Der Regler hat sich selbst über den Verlauf des ersten bis dritten Beatmungszyklus 51 bis 53 so gut an die Gegebenheiten der vorliegenden Beatmungssituation angepasst, dass ein effektives Nachführen des Beatmungsdrucks 28 entlang der Sollkurve möglich ist.

Obwohl die Erfindung mit Bezug auf beispielhafte Ausführungsformen beschrieben worden ist, ist es für einen Fachmann ersichtlich, dass verschiedene Änderungen vorgenommen und Äquivalente verwendet werden können, ohne den Bereich der Erfindung zu verlassen. Die Erfindung soll nicht durch die beschriebenen spezifischen Ausführungsformen beschränkt sein. Vielmehr enthält sie alle Ausführungsformen, die unter die angehängten Patentansprüche fallen.

**Patentansprüche**

1. System (2) zur Regelung einer Beatmungsgröße (28) eines Beatmungsgeräts (100) für die Nachführung entlang einer Sollkurve (16), aufweisend:

   mindestens einen Sensor (4), der eingerichtet ist, Momentanwerte (20) für die Beatmungsgröße (28) zu erfassen;
   einen Aktuator (6), der eingerichtet ist, die Beatmungsgröße (28) des Beatmungsgeräts (100) anzupassen;
   einen Speicher (8), in dem die Sollkurve (16) für die Beatmungsgröße hinterlegt ist, wobei die Sollkurve (16) für eine Inspirationsphase und/oder für eine Expirationsphase hinterlegt ist; und
   einen Regler (10), der zumindest ein Proportional-Glied und ein Integral-Glied aufweist, der eingerichtet ist, aus der Sollkurve (16) und den Momentanwerten (20) für die Beatmungsgröße (28) eine Regeldifferenz (14) zu bestimmen, und der eingerichtet ist, den Aktuator (6) zu steuern; wobei der Regler (10) eingerichtet ist, einen Verstärkungsfaktor des Integral-Glieds gemäß mindestens einem der folgenden Merkmale anzupassen:

   (i) Erhöhen des Verstärkungsfaktors des Integral-Glieds, wenn das Integral (30) der Regeldifferenz (24) für die Inspirationsphase und/oder die Expirationsphase einen vorbestimmten Integral-Schwellenwert (32) überschreitet;
   (ii) Reduzieren des Verstärkungsfaktors des Integral-Glieds, wenn eine Anzahl von Nulldurchgängen (41, 42, 43, 44, 45, 46) der Regeldifferenz (24) während der Inspirationsphase und/oder während der Expirationsphase einen vorbestimmten Nulldurchgang-Schwellenwert überschreitet.

2. System (2) nach Anspruch 1,

   wobei die Beatmungsgröße (28) eines von Beatmungsdruck und Beatmungsvolumen ist; und/oder
   wobei der Aktuator (6) einen gesteuerten Bläser und/oder ein gesteuertes Ventil aufweist.

3. System (2) nach einem der vorhergehenden Ansprüche, wobei der Regler (10) ein Proportional-Integral-Regler (PI-Regler) ist.

4. System (2) nach einem der vorhergehenden Ansprüche, wobei der Regler (10) eingerichtet ist, einen Verstärkungsfaktor des Proportional-Glieds unverändert zu lassen, wenn der Verstärkungsfaktor des Integral-Glieds angepasst wird, wobei der Regler (10) insbesondere eingerichtet ist, den Verstärkungsfaktor des Proportional-Glieds im Betrieb generell unverändert zu lassen.

5. System (2) nach einem der vorhergehenden Ansprüche,

   wobei der Regler (10) eingerichtet ist, den Verstärkungsfaktor des Integral-Glieds auf einen Initialwert zu setzen;
   wobei der Initialwert insbesondere abhängig von mindestens einem Patientenparameter, wie z.B. Alter oder Gewicht, ist, und/oder
   wobei der Regler (10) insbesondere eingerichtet ist, den Verstärkungsfaktor des Integral-Glieds einmalig bei Betriebsbeginn auf den Initialwert zu setzen, oder insbesondere eingerichtet ist, den Verstärkungsfaktor des Integral-Glieds in vorbestimmten Abständen auf den Initialwert zu setzen.

6. System (2) nach einem der vorhergehenden Ansprüche, wobei der Regler (10) eingerichtet ist, den Verstärkungsfaktor des Integral-Glieds für die Nachführung entlang der Sollkurve (16) für die momentane Inspirationsphase bzw. die momentane Expirationsphase anzupassen.

7. System (2) nach einem der vorhergehenden Ansprüche, wobei der Regler (10) eingerichtet ist, das Integral (30) der Regeldifferenz (24) bei einem Nulldurchgang der Regeldifferenz auf null zu setzen.

8. System (2) nach einem der vorhergehenden Ansprüche, wobei eine Mehrzahl von Integral-Schwellenwerten vorgesehen ist und wobei der Regler eingerichtet ist, den Verstärkungsfaktor des Integral-Glieds mehrfach zu erhöhen, wenn das Integral (30) der Regeldifferenz (24) mehrere der Mehrzahl von Integral-Schwellenwerten überschreitet.

9. System (2) nach einem der vorhergehenden Ansprüche, wobei eine Mehrzahl von Nulldurchgang-Schwellenwerten vorgesehen ist und wobei der Regler (10) eingerichtet ist, den Verstärkungsfaktor des Integral-Glieds mehrfach zu reduzieren, wenn die Anzahl von Nulldurchgängen mehrere der Mehrzahl von Nulldurchgang-Schwellenwerten überschreitet.

10. System (2) nach einem der vorhergehenden Ansprüche,

    wobei der Regler (10) eingerichtet ist, den Verstärkungsfaktor des Integral-Glieds in Merkmal (i) mit einem ersten Delta-Wert zu erhöhen, und/oder wobei der Regler (10) eingerichtet ist, den Verstärkungsfaktor des Integral-Glieds in Merkmal (ii) um einen zweiten Delta-Wert zu reduzieren, wobei insbesondere der erste Delta-Wert von dem zweiten Delta-Wert verschieden ist.

11. System (2) nach einem der vorhergehenden Ansprüche, wobei der Regler (10) weiterhin eingerichtet ist, den Verstärkungsfaktor des Integral-Glieds gemäß dem folgenden Merkmal anzupassen:

    (iii) Reduzieren des Verstärkungsfaktors des Integral-Glieds, wenn der Momentanwert (20) für die Beatmungsgröße (28) die Sollkurve (16) um einen Sicherheits-Schwellenwert (48) übersteigt, wobei der Sicherheits-Schwellenwert (48) insbesondere zwischen 1,5 mbar und 3 mbar liegt.

12. Beatmungsgerät (100), aufweisend ein System (2) zur Regelung einer Beatmungsgröße (28) nach einem der vorhergehenden Ansprüche.

13. Beatmungsgerät (100) nach Anspruch 12, weiterhin aufweisend:

    ein Verbindungselement (130, 140), insbesondere einen einlumigen oder zweilumigen Verbindungsschlauch, und ein Patientenapplikationsstück (150), insbesondere einen Tubus oder eine Beatmungsmaske, wobei das Patientenapplikationsstück (150) an einem Patienten-seitigen Endbereich des Verbindungselements (130, 140) angeschlossen ist.

14. Beatmungsgerät (100) nach Anspruch 13, wobei der Sensor (4) an einem Beatmungsgerät-seitigen Endbereich des Verbindungselements (130, 140) angeordnet ist oder wobei der Sensor (4) im Bereich des Anschlusses zwischen Verbindungselement (130, 140) und Patientenapplikationsstück (150) angeordnet ist oder wobei ein erster Sensor an einem Beatmungsgerät-seitigen Endbereich des Verbindungselements (130, 140) und ein zweiter Sensor im Bereich des Anschlusses zwischen Verbindungselement (130, 140) und Patientenapplikationsstück (150) angeordnet sind.

15. Verfahren zur Regelung einer Beatmungsgröße (28) eines Beatmungsgeräts (100) für die Nachführung entlang einer Sollkurve (16), aufweisend:

    Erfassen von Momentanwerten (20) für die Beatmungsgröße (28);
    Bestimmen einer Regeldifferenz (24) aus den Momentanwerten (20) für die Beatmungsgröße (28) und der Sollkurve (16) für die Beatmungsgröße (28), wobei die Sollkurve (16) für eine Inspirationsphase und/oder für eine Expirationsphase vorgegeben ist;
    Bestimmen einer Steuergröße (26) für einen Aktuator (6), wobei das Bestimmen der Steuergröße (26) gemäß einer Regelfunktion (18) erfolgt, welche zumindest ein Proportional-Glied und ein Integral-Glied aufweist;
    Anpassen des Integral-Glieds der Regelfunktion (18) gemäß mindestens einem der folgenden Merkmale:

        (i) Erhöhen des Verstärkungsfaktors des Integral-Glieds, wenn das Integral (30) der Regeldifferenz (24) für die Inspirationsphase und/oder die Expirationsphase einen vorbestimmten Integral-Schwellenwert (32) überschreitet;
        (ii) Reduzieren des Verstärkungsfaktors des Integral-Glieds, wenn eine Anzahl von Nulldurchgängen (41, 42, 43, 44, 45, 46) der Regeldifferenz (24) während der Inspirationsphase und/oder während der Expirationsphase einen vorbestimmten Nulldurchgang-Schwellenwert überschreitet;

    und
    Steuern des Aktuators (6) gemäß der bestimmten Steuergröße (26) zum Anpassen der Beatmungsgröße (28) des Beatmungsgeräts (100).

16. Computerprogramm, welches Programmanweisungen enthält, die bei Ausführung auf einer Datenverarbeitungsanlage ein Verfahren nach Anspruch 15 durchführen.

**Claims**

1. A system (2) for controlling a ventilation variable (28) of a ventilation device (100) for tracking along a target curve (16), comprising:

    at least one sensor (4) which is adapted to detect instantaneous values (20) for the ventilation variable (28);

an actuator (6) which is adapted to adjust the ventilation variable (28) of the ventilation device (100);

a memory (8) in which the target curve (16) for the ventilation variable is stored, the target curve (16) being stored for an inspiration phase and/or for an expiration phase; and

a controller (10) which has at least a proportional term and an integral term and which is adapted to determine a control difference (14) from the target curve (16) and the instantaneous values (20) for the ventilation variable (28), and which is adapted to control the actuator (6);

wherein the controller (10) is adapted to adjust a gain factor of the integral term according to at least one of the following features:

(i) increasing the gain factor of the integral term when the integral (30) of the control difference (24) for the inspiration phase and/or the expiration phase exceeds a predetermined integral threshold value (32);

(ii) reducing the gain factor of the integral term when a number of zero crossings (41, 42, 43, 44, 45, 46) of the control difference (24) during the inspiration phase and/or during the expiration phase exceeds a predetermined zero-crossing threshold value.

2. The system (2) according to claim 1,

wherein the ventilation variable (28) is one of ventilation pressure and ventilation volume; and/or

wherein the actuator (6) comprises a controlled blower and/or a controlled valve.

3. The system (2) according to any of the preceding claims,

wherein the controller (10) is a proportional integral controller (PI controller).

4. The system (2) according to any of the preceding claims,

wherein the controller (10) is adapted to leave a gain factor of the proportional term unchanged when the gain factor of the integral term is adjusted,

wherein the controller (10) is adapted in particular to leave the gain factor of the proportional term generally unchanged during operation.

5. The system (2) according to any of the preceding claims,

wherein the controller (10) is adapted to set the gain factor of the integral term to an initial value;

wherein the initial value in particular is dependent on at least one patient parameter, such as e.g. age or weight;

and/or

wherein the controller (10) is adapted in particular to set the gain factor of the integral term to the initial value once at the start of operation, or is adapted in particular to set the gain factor of the integral term to the initial value in predetermined intervals.

6. The system (2) according to any of the preceding claims,

wherein the controller (10) is adapted to adjust the gain factor of the integral term for tracking along the target curve (16) for the current inspiration phase or the current expiration phase.

7. The system (2) according to any of the preceding claims,

wherein the controller (10) is adapted to set the integral (30) of the control difference (24) to zero at a zero crossing of the control difference.

8. The system (2) according to any of the preceding claims,

wherein a plurality of integral threshold values is provided and wherein the controller is adapted to increase the gain factor of the integral term a plurality of times when the integral (30) of the control difference (24) exceeds several of the plurality of integral threshold values.

9. The system (2) according to any of the preceding claims,

wherein a plurality of zero-crossing threshold values is provided, and wherein the controller (10) is adapted to reduce the gain factor of the integral term a plurality of times when the number of zero crossings exceeds several of the plurality of zero-crossing threshold values.

10. The system (2) according to any of the preceding claims,

wherein the controller (10) is adapted to increase the gain factor of the integral term in feature (i) with a first delta value, and/or wherein the controller (10) is adapted to reduce the gain factor of the integral term in feature (ii) by a second delta value,

wherein in particular the first delta value is different from the second delta value.

11. The system (2) according to any of the preceding claims,

wherein the controller (10) is further adapted to adjust the gain factor of the integral term according to the following feature:

(iii) reducing the gain factor of the integral term when the instantaneous value (20) for the ventilation variable (28) exceeds the target curve (16) by a safety threshold value (48), wherein the safety threshold value (48) in particular is between 1.5 mbar and 3 mbar.

12. A ventilation device (100), comprising a system (2) for controlling a ventilation variable (28) according to any of the preceding claims.

13. The ventilation device (100) according to claim 12, further comprising:

a connecting member (130, 140), in particular a single-lumen or double-lumen connecting hose, and a patient application piece (150), in particular a tube or a respiratory mask, wherein the patient application piece (150) is connected to a patient-side end portion of the connecting member (130, 140).

14. The ventilation device (100) according to claim 13, wherein the sensor (4) is arranged at a ventilation device-side end portion of the connecting member (130, 140) or wherein the sensor (4) is arranged in the region of the connection between connecting member (130, 140) and patient application piece (150) or wherein a first sensor is arranged at a ventilation device-side end portion of the connecting member (130, 140) and a second sensor is arranged in the region of the connection between connecting member (130, 140) and patient application piece (150).

15. A method of controlling a ventilation variable (28) of a ventilation device (100) for tracking along a target curve (16), comprising the steps of:

acquiring instantaneous values (20) for the ventilation variable (28); determining a control difference (24) from the instantaneous values (20) for the ventilation variable (28) and the target curve (16) for the ventilation variable (28), the target curve (16) being predetermined for an inspiration phase and/or for an expiration phase; determining a control variable (26) for an actuator (6), wherein determining the control variable (26) is performed according to a control function (18) having at least a proportional term and an integral term; adjusting the integral term of the control function (18) according to at least one of the following features:

(i) increasing the gain factor of the integral term when the integral (30) of the control difference (24) for the inspiration phase and/or the expiration phase exceeds a predetermined integral threshold value (32); (ii) reducing the gain factor of the integral term when a number of zero crossings (41, 42, 43, 44, 45, 46) of the control difference (24) during the inspiration phase and/or during the expiration phase exceeds a predetermined zero-crossing threshold value;

and controlling the actuator (6) according to the determined control variable (26) for adjusting the ventilation variable (28) of the ventilation device (100).

16. A computer program containing program instructions which, when executed on a data processing system, perform a method according to claim 15.

**Revendications**

1. Système (2) de régulation d'une grandeur de ventilation (28) d'un appareil de ventilation (100) pour le suivi le long d'une courbe de consigne (16), présentant:

au moins un capteur (4) qui est conçu pour détecter des valeurs instantanées (20) pour la grandeur de ventilation (28) ; un actionneur (6) qui est conçu pour adapter la grandeur de ventilation (28) de l'appareil de ventilation (100) ; une mémoire (8) dans laquelle est enregistrée la courbe de consigne (16) pour la grandeur de ventilation, la courbe de consigne (16) étant enregistrée pour une phase d'inspiration et/ou pour une phase d'expiration ; et un régulateur (10) qui présente au moins un élément proportionnel et un élément intégral, qui est conçu pour déterminer une différence de régulation (14) à partir de la courbe de consigne (16) et des valeurs instantanées (20) pour la grandeur de ventilation (28), et qui est conçu pour commander l'actionneur (6) ; dans lequel le régulateur (10) est configuré pour adapter un facteur d'amplification de l'élément intégral selon au moins l'une des caractéristiques suivantes :

(i) augmentation du facteur d'amplification de l'élément intégral lorsque l'intégrale (30) de la différence de régulation (24) pour la phase d'inspiration et/ou la phase d'expiration dépasse une valeur seuil d'intégrale prédéterminée (32) ;

(ii) réduction du facteur d'amplification de l'élément intégral lorsqu'un nombre de passages par zéro (41, 42, 43, 44, 45, 46) de la différence de régulation (24) pendant la phase d'inspiration et/ou pendant la phase d'expiration dépasse un seuil de passage par zéro prédéterminé.

2. Le système (2) selon la revendication 1,

dans lequel la grandeur de ventilation (28) est l'une parmi la pression de ventilation et du volume de ventilation ;
et/ou
dans lequel l'actionneur (6) comporte un ventilateur commandé et/ou une vanne commandée.

3. Le système (2) selon l'une quelconque des revendications précédentes, dans lequel le régulateur (10) est un régulateur proportionnel-intégral (régulateur PI).

4. Le système (2) selon l'une quelconque des revendications précédentes, dans lequel le régulateur (10) est conçu pour laisser inchangé un facteur d'amplification de l'élément proportionnel lorsque le facteur d'amplification de l'élément intégral est adapté, le régulateur (10) étant notamment conçu pour laisser le facteur d'amplification de l'élément proportionnel généralement inchangé pendant le fonctionnement.

5. Le système (2) selon l'une quelconque des revendications précédentes,

dans lequel le régulateur (10) est agencé pour régler le facteur d'amplification de l'élément intégral sur une valeur initiale ;
dans lequel la valeur initiale dépend notamment d'au moins un paramètre du patient, tel que l'âge ou le poids,
et/ou
dans lequel le régulateur (10) est notamment conçu pour régler le facteur d'amplification de l'élément intégral une seule fois à la valeur initiale au début du fonctionnement, ou est en particulier conçu pour régler le facteur d'amplification de l'élément intégral à la valeur initiale à des intervalles prédéfinis.

6. Le système (2) selon l'une quelconque des revendications précédentes, dans lequel le régulateur (10) est conçu pour ajuster le facteur d'amplification de l'élément intégral pour le suivi le long de la courbe de consigne (16) pour la phase d'inspiration momentanée ou la phase d'expiration momentanée.

7. Le système (2) selon l'une quelconque des revendications précédentes, dans lequel le régulateur (10) est conçu pour mettre à zéro l'intégrale (30) de la différence de régulation (24) lors d'un passage par zéro de la différence de régulation.

8. Le système (2) selon l'une quelconque des revendications précédentes, dans lequel une pluralité de valeurs de seuil intégrales sont prévues et dans lequel le régulateur est agencé pour augmenter plusieurs fois le facteur d'amplification de l'élément intégral lorsque l'intégrale (30) de la différence de régulation (24) dépasse plusieurs de la pluralité de valeurs de seuil intégrales.

9. Le système (2) selon l'une quelconque des revendications précédentes, dans lequel une pluralité de valeurs de seuil de passage par zéro est prévue et dans lequel le régulateur (10) est adapté pour réduire le facteur d'amplification de l'élément intégral plusieurs fois lorsque le nombre de passages par zéro dépasse plusieurs de la pluralité de valeurs de seuil de passage par zéro.

10. Le système (2) selon l'une quelconque des revendications précédentes,
dans lequel le régulateur (10) est agencé pour augmenter le facteur d'amplification de l'élément intégral dans la caractéristique (i) avec une première valeur delta, et/ou dans lequel le régulateur (10) est agencé pour réduire le facteur d'amplification de l'élément intégral dans la caractéristique (ii) d'une seconde valeur delta, la première valeur delta étant notamment différente de la seconde valeur delta.

11. Le système (2) selon l'une quelconque des revendications précédentes, dans lequel le régulateur (10) est en outre agencé pour ajuster le facteur d'amplification de l'élément intégral en fonction de la caractéristique suivante :
(iii) réduction du facteur d'amplification de l'élément intégral lorsque la valeur instantanée (20) pour la grandeur de ventilation (28) dépasse la courbe de consigne (16) d'une valeur seuil de sécurité (48), la valeur seuil de sécurité (48) étant notamment comprise entre 1,5 mbar et 3 mbar.

12. Un appareil respiratoire (100), comprenant un système (2) de régulation d'une grandeur de ventilation (28) selon l'une des revendications précédentes.

13. L'appareil respiratoire (100) selon la revendication 12, comprenant en outre :

un élément de raccordement (130, 140), en particulier un tube de raccordement à une ou deux lumières, et

une pièce d'application au patient (150), en particulier un tube ou un masque respiratoire, la pièce d'application au patient (150) étant raccordée à une partie d'extrémité côté patient de l'élément de raccordement (130, 140).

14. L'appareil respiratoire (100) selon la revendication 13, dans lequel le capteur (4) est disposé sur une zone d'extrémité de l'élément de raccordement (130, 140) située du côté du respirateur ou dans lequel le capteur (4) est disposé dans la zone de raccordement entre l'élément de raccordement (130, 140) et la pièce d'application au patient (150) ou dans lequel un premier capteur est disposé sur une zone d'extrémité de l'élément de raccordement (130, 140) située du côté du respirateur et un second capteur est disposé dans la zone de raccordement entre l'élément de raccordement (130, 140) et la pièce d'application au patient (150).

15. Procédé de régulation d'une grandeur de ventilation (28) d'un appareil respiratoire (100) pour le suivi le long d'une courbe de consigne (16), comprenant les étapes suivantes :

Saisie de valeurs instantanées (20) pour la grandeur de ventilation (28) ;

Détermination d'une différence de régulation (24) à partir des valeurs instantanées (20) pour la grandeur de ventilation (28) et de la courbe de consigne (16) pour la grandeur de ventilation (28), la courbe de consigne (16) étant prédéfinie pour une phase d'inspiration et/ou pour une phase d'expiration ;

Détermination d'une grandeur de commande (26) pour un actionneur (6), la détermination de la grandeur de commande (26) s'effectuant selon une fonction de régulation (18) qui présente au moins un élément proportionnel et un élément intégral ;

Adaptation de l'élément intégral de la fonction de régulation (18) selon au moins l'une des caractéristiques suivantes :

(i) augmentation du facteur d'amplification de l'élément intégral lorsque l'intégrale (30) de la différence de régulation (24) pour la phase d'inspiration et/ou la phase d'expiration dépasse une valeur seuil d'intégrale prédéterminée (32) ;

(ii) réduction du facteur d'amplification de l'élément intégral lorsqu'un nombre de passages par zéro (41, 42, 43, 44, 45, 46) de la différence de régulation (24) pendant la phase d'inspiration et/ou pendant la phase d'expiration dépasse un seuil de passage par zéro prédéterminé ;

et

Commande de l'actionneur (6) en fonction de la grandeur de commande déterminée (26) pour adapter la grandeur de ventilation (28) de l'appareil respiratoire (100).

16. Programme informatique comprenant des instructions de programme qui, lorsqu'elles sont exécutées sur un système de traitement de données, mettent en oeuvre un procédé selon la revendication 15.

# Fig. 1

Fig. 2

Fig. 3A

Fig. 3B

Fig. 4A

Fig. 4B

Fig. 5A

Fig. 5B

## Fig. 6A

## Fig. 6B

EP 4 003 472 B1

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 2005051469 A1
- WO 2016149743 A1